(19)

Europäisches
Patentamt
European
Patent Office
Office européen
des brevets

(11) **EP 4 450 647 A2**

(12) **EUROPEAN PATENT APPLICATION**

(43) Date of publication:
**23.10.2024   Bulletin 2024/43**

(21) Application number: **24153672.1**

(22) Date of filing: **24.01.2024**

(51) International Patent Classification (IPC):
***C12Q 1/6883*** (2018.01)     ***C12Q 1/689*** (2018.01)
***G16B 20/00*** (2019.01)

(52) Cooperative Patent Classification (CPC):
**C12Q 1/689; C12Q 1/6883; G16B 20/00;**
C12Q 2600/118; C12Q 2600/158

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:   **19.04.2023   IN 202321028612**

(71) Applicant: **Tata Consultancy Services Limited
Maharashtra (IN)**

(72) Inventors:
• **MERCHANT, MITALI**
   **411013 Pune, Maharashtra (IN)**
• **NAGPAL, SUNIL**
   **411013 Pune, Maharashtra (IN)**

• **HAQUE, MOHAMMED MONZOORUL**
   **411013 Pune, Maharashtra (IN)**
• **MANDE, SHARMILA SHEKHAR**
   **411013 Pune, Maharashtra (IN)**
• **PINNA, NISHAL KUMAR**
   **411013 Pune, Maharashtra (IN)**

(74) Representative: **Goddar, Heinz J.
Boehmert & Boehmert
Anwaltspartnerschaft mbB
Pettenkoferstrasse 22
80336 München (DE)**

Remarks:
The complete document including Reference
Table(s) and the Sequence Listing(s) can be
downloaded from the EPO website

(54) **EARLY RISK ASSESSMENT OF PRETERM DELIVERY IN A SUBJECT**

(57)    This disclosure relates more particularly to risk assessment of preterm delivery (PTD) in the subject and designing a personalized recommendation for the same. Conventional techniques for PTD risk assessment are either invasive or minimally invasive and leaves little time for subjects to take precautionary or corrective medical advice or procedures to reduce or obviate the risk. The present disclosure provides the risk assessment of the PTD, by quantifying a microbial abundance in oral or gut microbiome for a pregnant woman, identifying a certain combination of microbial biomarkers using an ensemble of models for accurate risk assessment of the PTD and subsequently suggesting a personalized recommendation for at risk subject. The present assessment technique is completely non-invasive and further helps in characterizing the risk of the PTD.

FIG. 1

EP 4 450 647 A2

Processed by Luminess, 75001 PARIS (FR)

**Description**

CROSS-REFERENCE TO RELATED APPLICATIONS AND PRIORITY

The present application claims priority to Indian application no. 202321028612, filed on April 19, 2023.

TECHNICAL FIELD

The disclosure herein generally relates to risk assessment of disorders present in a subject and, more particularly, to method and system for an early risk assessment of preterm delivery in a subject and designing a personalized recommendation for the same.

BACKGROUND

Preterm delivery (PTD) is the birth of a baby (an infant) at fewer than 37 weeks gestational age, as opposed to full-term delivery at approximately 40 weeks which is the standard delivery period. The cause of spontaneous preterm delivery is often not known and due to various reasons but mostly related to unhealthy status or medical complications in the pregnant women. The preterm delivery (PTD) to infants may pose greater risks including but are not limited to cerebral palsy, delays in development, hearing problems and problems with their vision. The earlier a baby is born, the greater these risks will be. An estimated 15 million babies are born preterm globally every year and almost a million of them die due to complications of preterm birth.

The PTD may be prevented in those at risk by detecting and providing with suitable treatment. Hence, the PTD of the infants to be detected in pregnant women as early as possible to avoid the risks of possible disorders for the infants. Conventional techniques for PTD risk assessment are either invasive or minimally invasive (for example, using vaginal swabs). Further, existing PTD risk assessment or screening procedures provide diagnostic results of practical utility only in the 'later' stages of the delivery period (i.e., in the late second trimester or in the third or last trimester). This leaves little time for subjects to take precautionary or corrective medical advice or procedures to reduce or obviate the risk.

SUMMARY

Embodiments of the present disclosure present technological improvements as solutions to one or more of the above-mentioned technical problems recognized by the inventors in conventional systems.

In an aspect, a method for an early risk assessment of preterm delivery in a subject is provided. The method comprising the steps of: collecting a biological sample from the subject whose risk of preterm delivery is to be assessed; extracting microbial deoxyribonucleic acid (DNA) sequences from the biological sample; determining a quantitative abundance of each of a plurality of predetermined microbial marker sequences associated with the biological sample, from the extracted DNA sequences, using a set of probes specific to each of the plurality of predetermined microbial marker sequences associated with the biological sample, through a multiplexed quantitative Polymerase Chain Reaction (qPCR) technique; determining a model score based on the quantitative abundance of each of the plurality of predetermined microbial marker sequences associated with the biological sample, using a pre-determined machine learning (ML) model associated to the biological sample; performing the early risk assessment of preterm delivery in the subject, based on the model score and a predefined threshold value associated with the biological sample; and designing, a personalized recommendation for the subject assessed as having risk of preterm delivery, by utilizing a set of rules for the plurality of predetermined microbial marker sequences that constitute the pre-determined machine learning model to identify one or more personalized antibiotic target candidates that ameliorate the risk of preterm delivery.

In yet another aspect, a kit for an early risk assessment of preterm delivery in a subject is provided. The kit comprising: an input module for receiving a biological sample from the subject whose risk of preterm delivery is to be assessed, wherein the biological sample of the subject is one of: (i) a stool sample and (ii) a saliva sample; one or more hardware processors configured to analyze the biological sample using the method; and an output module for displaying the early risk assessment of preterm delivery in the subject, based on the analysis of the one or more hardware processors.

In an embodiment, the biological sample collected from the subject is one of: (i) a stool sample and (ii) a saliva sample.

In an embodiment, the plurality of predetermined microbial marker sequences associated with the biological sample being the stool sample are listed in Table 1 comprising Gut_seq1 to Gut_seq15.

In an embodiment, the plurality of predetermined microbial marker sequences associated with the biological sample being the saliva sample are listed in Table 2 comprising Sal_seq1 to Sal_seq9.

In an embodiment, the set of probes specific to each of the plurality of predetermined microbial marker sequences associated with the biological sample being the stool sample are utilized in a first multiplexed qPCR run, a second multiplexed qPCR run, a third multiplexed qPCR run, a fourth multiplexed qPCR run, and a fifth multiplexed qPCR run, to determine the quantitative abundance of each of the plurality of predetermined microbial marker sequences associated with the biological sample, and wherein: the plurality of predetermined microbial marker sequences, the quantitative abundance of which are being determined through the first multiplexed qPCR run are: Gut_seq1, Gut_seq2, Gut_seq3, and Gut_seq4 listed in Table 1; the plurality of predetermined microbial marker sequences, the quantitative abundance of which are being determined through the second multiplexed qPCR run are: Gut_seq1, Gut_seq5, Gut_seq6, and Gut_seq7 listed in Table 1; the plurality of predetermined microbial marker sequences, the quantitative abundance of which are being determined through the third multiplexed qPCR run are: Gut_seq8, Gut_seq5, Gut_seq9, and Gut seq10 listed in Table 1; the plurality of predetermined microbial marker sequences, the quantitative abundance of which are being determined through the fourth multiplexed qPCR run are: Gut_seq8, Gut_seq11, Gut_seq12, and Gut_seq13 listed in Table 1; and the plurality of predetermined microbial marker sequences, the quantitative abundance of which are being determined through the fifth multiplexed qPCR run are: Gut_seq6, Gut_seq11, Gut_seq14, and Gut_seq15 listed in Table 1.

In an embodiment, the set of probes specific to each of the plurality of predetermined microbial marker sequences associated with the biological sample being the saliva sample are utilized in a sixth multiplexed qPCR run, a seventh multiplexed qPCR run, and an eighth multiplexed qPCR run, to determine the quantitative abundance of each of the plurality of predetermined microbial marker sequences associated with the biological sample, and wherein: the plurality of predetermined microbial marker sequences, the quantitative abundance of which are being determined through the sixth multiplexed qPCR run are: Sal_seq1, Sal_seq2, Sal_seq3, and Sal_seq4 listed in Table 2; the plurality of predetermined microbial marker sequences, the quantitative abundance of which are being determined through the seventh multiplexed qPCR run are: Sal_seq4, Sal_seq2, Sal_seq5, and Sal_seq6 listed in Table 2; and the plurality of predetermined microbial marker sequences, the quantitative abundance of which are being determined through the eighth multiplexed qPCR run are: Sal_seq7, Sal_seq8, Sal_seq5, and Sal_seq9 listed in Table 2.

In an embodiment, the pre-determined machine learning (ML) model associated to the biological sample is an ensemble ML model that is built using a microbial marker sequence abundance data associated to a plurality of training biological samples.

In an embodiment, the plurality of predetermined microbial marker sequences associated with the biological sample are features of the associated pre-determined machine learning (ML) model.

In an embodiment, one or more predetermined microbial marker sequences out of the plurality of predetermined microbial marker sequences associated with the biological sample being the stool sample, are common to one or more of the first multiplexed qPCR run, the second multiplexed qPCR run, the third multiplexed qPCR run, the fourth multiplexed qPCR run, and the fifth multiplexed qPCR run for determining the quantitative abundance, and wherein the one or more predetermined microbial marker sequences that are common to the one or more of the first multiplexed qPCR run, the second multiplexed qPCR run, the third multiplexed qPCR run, the fourth multiplexed qPCR run, and the fifth multiplexed qPCR run are determined based on (i) a median abundance of each of the plurality of predetermined microbial marker sequences obtained from the associated plurality of training biological samples, and (ii) a frequency of occurrence of each of the plurality of predetermined microbial marker sequences constituting the associated ensemble ML model.

In an embodiment, one or more predetermined microbial marker sequences out of the plurality of predetermined microbial marker sequences associated with the biological sample being the saliva sample, are common to one or more of the sixth multiplexed qPCR run, the seventh multiplexed qPCR run, and the eighth multiplexed qPCR run for determining the quantitative abundance, and wherein the one or more predetermined microbial marker sequences that are common to the one or more of the sixth multiplexed qPCR run, the seventh multiplexed qPCR run, and the eighth multiplexed qPCR run are determined based on (i) a median abundance of each of the plurality of predetermined microbial marker sequences obtained from the associated plurality of training biological samples, and (ii) a frequency of occurrence of each of the plurality of predetermined microbial marker sequences constituting the associated ensemble ML model.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary block diagram of a system for an early risk assessment of preterm delivery in a subject, according to some embodiments of the present disclosure.
FIGS. 2A and 2B are flowcharts illustrating a method for an early risk assessment of preterm delivery in a subject, according to some embodiments of the present disclosure.

FIG. 3A illustrates an exemplary probe and multiplexed qPCR design for detecting and determining the quantitative abundance of each of a plurality of predetermined microbial marker sequences associated with the stool sample, according to some embodiments of the present disclosure.

FIG. 3B illustrates an exemplary probe and multiplexed qPCR design for detecting and determining the quantitative abundance of each of the plurality of predetermined microbial marker sequences associated with the saliva sample, according to some embodiments of the present disclosure.

FIG. 4A, 4B and 4C are flowcharts illustrating steps involved in building a pre-determined machine learning model according to some embodiments of the present disclosure.

FIG. 5 illustrates an exemplary block diagram of a kit for an early risk assessment of preterm delivery in a subject, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Technological advances in medical diagnostics and therapeutics in the last decade have greatly reduced the burden of several life-threatening diseases affecting young children. The Millennium Development Goals (MDG) report released by United Nations (UN) in 2015 indicates significant reduction in the incidence rates of malaria, tuberculosis, measles, and AIDS in the last 15 years. However, amidst these encouraging signs of improvement, the report highlights that a majority of deaths in children (under 5) occur within the first 28 days of life (i.e., the neonatal period). Complications arising due to preterm births are indicated as the single largest contributor to neonatal deaths. Surprisingly, in comparison to the decrease in incidence rates of other diseases across the globe, the rate of preterm births has remained more or less constant, irrespective of a country's economic status. For instance, in 2016, the rate of preterm births in US stood at 9.85%, which is quite comparable to the rate (~12%) tracked in countries from the developing world (such as in India, Nigeria, etc.)

An estimated 15 million babies are born preterm every year globally and almost a million of them die due to complications of the preterm birth. The situation in India and other developing countries is particularly alarming. As a country, India accounts for nearly 24% of the global burden of preterm births. With approximately 1 in 8 babies being preterm, India has the highest number of reported annual preterm births in the world with an associated mortality rate of approximately 10%. In terms of numbers, approximately 3.5 million babies are born preterm every year in India. Of them, nearly 10% (~3.2 Lakh) die due to preterm birth related complications.

In normal pregnancies (delivery), the overall period of gestation typically ranges between 38-42 weeks. A delivery (with "live" singleton or multiple childbirths) is considered 'preterm' if the duration of pregnancy falls below 37 completed weeks of gestation i.e., 259 days since the first day of a woman's last menstrual period. By definition, the preterm delivery (PTD) is childbirth that occurs between the date of fetal viability and the end of the 37th week of gestation. The fetal viability is defined as the potential of the fetus to survive outside the uterus after birth, natural or induced. The fetal viability is usually placed at about seven months (28 completed weeks) of gestation, but in some cases, the fetal viability may occur earlier, even between 20-24 completed weeks of gestation. Preterm labor begins with contractions of the uterus before 37 weeks of pregnancy that cause the cervix to thin out and open up. If the preterm labor cannot be stopped, it leads to the preterm birth.

The preterm births are further subdivided based on gestational age (GA):

- Extremely preterm (GA <28 weeks)
- Very preterm (GA 28 to <32 weeks)
- Moderate or late preterm (GA 32 to <37 completed weeks of gestation)

Of all preterm births, moderate or late preterm births account for 84.3%, very preterm 10.4% and extremely preterm about 5.2%. It is important to note here that there exists a distinct difference between a still birth and babies who are born extremely preterm. By definition, a still birth refers to baby delivered (at greater than equal to 28 completed weeks of gestation) with no signs of life. The still births are not to be confused with "miscarriages" in which fetal death occurs before 20 completed weeks. It is significant to note that the still births account for 5% of all preterm births.

Hence, identifying, quite early in pregnancy or delivery period (ideally the first three months), the risk of a preterm delivery (PTD) outcome, can help caretakers or doctors to take suitable precautions and employ additional measures of care for prolonging the gestation period towards a normal term period. Various diagnostic or risk-assessment markers (physical and/or biochemical) are present that can accurately indicate, at an early stage of pregnancy, the risk or possibility of progression towards a preterm delivery outcome assume a lot of significance.

There are certain diagnostic or assessment techniques in the art that can be employed at various stages of the pregnancy period for predicting the PTD outcome. Although some of these techniques have some diagnostic value in the first trimester of pregnancy, the low sensitivity renders them unsuitable for practical usage. Conventional physical or biochemical diagnostic markers have relatively higher or better predictive ability, but mostly suited for application at mid or late pregnancy stages. Although such predictive or diagnostic ability aids in the process of relocating the woman to a suitable medical setting, the short duration between diagnosis and delivery makes it difficult for adopting meaningful intervention strategies that can promote towards full-term gestation. This highlights the need for markers that can predict, at a very early stage of pregnancy (ideally in the first trimester), the possibility of the pregnancy leading to a preterm delivery outcome.

Furthermore, many existing diagnostics of predictive techniques used for assessing the risk of the PTD outcome rely on the subjects (a) presenting themselves with vaginal infections and/ or (b) having abnormalities detected in radiological procedures and/ or (c) having the preterm history in earlier deliveries. This makes such diagnostic or predictive procedures unsuitable in cases of first pregnancies or preterm outcomes which are not driven by vaginal infections or fetal or uterine abnormalities which are also known as asymptomatic preterm delivery.

The present disclosure solves the challenges present in the existing state of art, for accurate risk assessment of the PTD delivery in the subject, by providing the early prediction (within 13 weeks of pregnancy or earlier) regarding risk or predisposition to PTD. This helps in providing sufficient time for pregnant women (detected with a high risk) to take required precautionary or corrective medical advice (such as taking progesterone supplements) or procedures (e.g., cervical suturing) that reduce or obviate the risk of the PTD. The present disclosure provides the scope of complete monitoring of a pregnant woman in the first and second trimester for identification of risk of the PTD. With the present disclosure, the woman can, at any point of the pregnancy period (<27 weeks), get an assessment of the risk of the PTD.

The present disclosure provides the risk assessment of the PTD, by quantifying a microbial abundance in oral or gut microbiome for a pregnant woman, identifying a certain combination of microbial biomarkers using an ensemble of models for accurate risk assessment of the PTD and subsequently designing a personalized recommendation for at risk subject. The present assessment technique is completely non-invasive and further helps in characterizing the risk of the PTD.

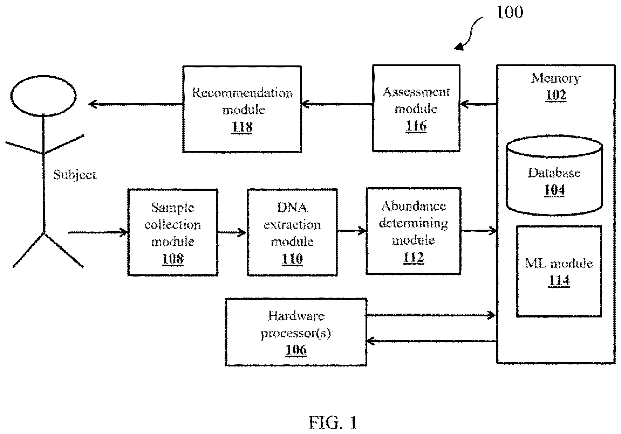

Referring now to the drawings, and more particularly to FIG. 1 through 5, where similar reference characters denote corresponding features consistently throughout the figures, there are shown preferred embodiments and these embodiments are described in the context of the following exemplary system and/or method.

FIG. 1 illustrates an exemplary block diagram of a system 100 for the early risk assessment of preterm delivery in a subject, according to some embodiments of the present disclosure. In an embodiment, the system 100 includes a memory 102, a database 104, one or more hardware processors 106, a sample collection module 108, a DNA extraction module 110, an abundance determining module 112, a machine learning (ML) module 114, an assessment module 116, and a recommendation module 118. In an embodiment, the database 104 and the machine learning (ML) module 114 are stored in the memory 102.

In an embodiment, the sample collection module 108 is configured to collect a biological sample of the subject whose risk of the PTD is to be assessed. The DNA extraction module 110 is configured to extract microbial deoxyribonucleic acid (DNA) sequences from the biological sample. The abundance determining module 112 is configured to determine a quantitative abundance of each of a plurality of predetermined microbial marker sequences associated with the biological sample, from the extracted DNA sequences.

The machine learning (ML) module 114 is configured to determine a model score based on the quantitative abundance of each of the plurality of predetermined microbial marker sequences associated with the biological sample. The assessment module 116 is configured to perform the early risk assessment of preterm delivery in the subject based on the model score. Lastly, the recommendation module 118 is configured to design a personalized recommendation for the subject if detected with the risk assessment of the PTD.

In an embodiment, the one or more hardware processors 106 can be implemented as one or more microprocessors, microcomputers, microcontrollers, digital signal processors, central processing units, state machines, logic circuitries, and/or any devices that manipulate signals based on operational instructions. Among other capabilities, the one or more hardware processors 106 is configured to fetch and execute computer-readable instructions stored in the memory 102. In an embodiment, the system 100 can be implemented in a variety of computing systems including laptop computers, notebooks, hand-held devices such as mobile phones, workstations, mainframe computers, servers, a network cloud and the like.

The memory 102 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes.

Further, the memory 102 may include a database 104 configured to include information regarding risk assessment of autism spectrum disorder present in the subject. The memory 102 may comprise information pertaining to input(s)/output(s) of each step performed by the one or more hardware processors 106 of the system 100 and methods of the present disclosure. In an embodiment, the database 104 may be external (not shown) to the system 100 and coupled to the system 100 via the I/O interfaces (not shown in FIG. 1).

In an embodiment, one or more data storage devices or the memory 102 operatively coupled to the one or more hardware processors 106. The system 100 with the one or more hardware processors 106 is configured to execute functions of one or more functional modules of the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

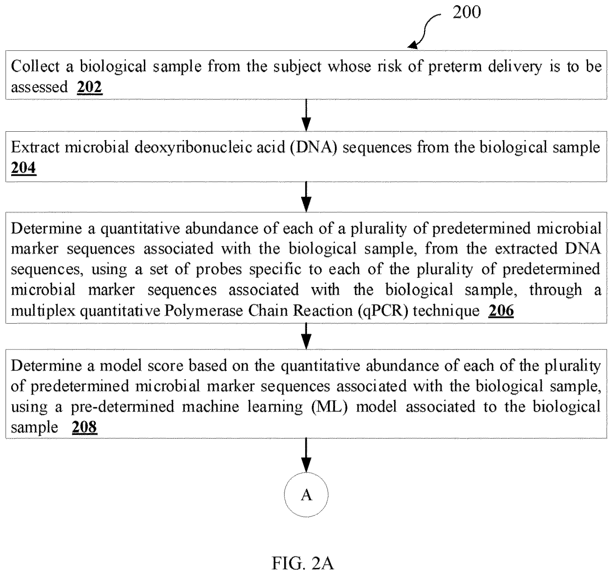

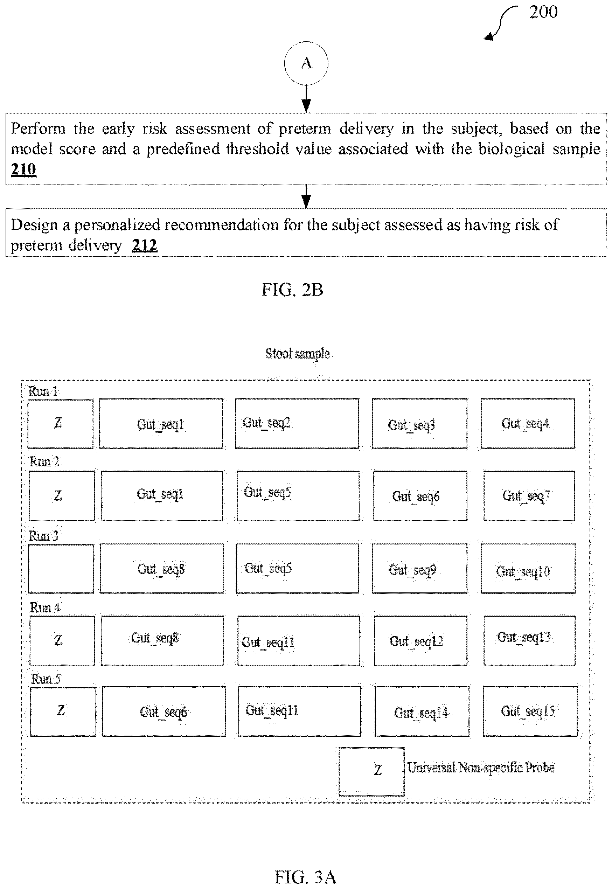

In an embodiment, the memory 102 comprises one or more data storage devices operatively coupled to the one or more hardware processors 106 and is configured to store instructions for execution of steps of the method depicted in FIGS. 2A and 2B by the one or more hardware processors 106. FIGS. 2A and 2B are flowcharts illustrating a method 200 for the early risk assessment of preterm delivery in the subject, according to some embodiments of the present disclosure. The steps of the method 200 of the present disclosure will now be explained with reference to the components or blocks of the system 100 as depicted in FIG. 1 and the steps of flow diagrams as depicted in FIGS. 2A and 2B. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of the method 200, the biological sample from the subject whose risk of preterm delivery is to be assessed, is collected through the sample collection module 108. In an embodiment, the subject or the individual is any pregnant women. The biological sample can be collected at any time during the pregnancy period or the delivery period in general. However, as the risk assessment of the PTD to be detected at the earliest possible, the biological sample can be collected particularly during the first or second trimesters of the pregnancy period.

In an embodiment, the biological sample is one of: (i) a stool sample and (ii) a saliva sample. Any one sample (or sub-sample) out the stool sample and the saliva sample from the subject is sufficient enough to assess the risk of the PTD in the subject according to the present disclosure. Both the stool sample and the saliva sample are site-specific samples and are completely non-invasive. In an embodiment, the saliva sample may refer to extracted salivary swabs or naturally out-flown saliva or voluntarily spitted saliva which is obtained in a non-stimulatory environment (where stimulations refer to behavioral or digestive triggers). In general, the saliva sample is extracted from the mouth site of the subject. The stool sample is a fecal sample obtained from the subject.

Further, at step 204 of the method 200, microbial deoxyribonucleic acid (DNA) sequences are extracted from the biological sample collected at step 202 of the method 200, through the DNA extraction module 110. In an embodiment, the extraction of microbial DNA sequences from the biological sample is performed by amplification of 16S rRNA marker genes (either full-length or specific variable regions of the gene) using a next-generation sequencing (NGS) platform, Oxford nanopore sequencing, or any other DNA sequencing technique and a platform (including a classical Sanger sequencing). In another embodiment, the NGS platforms include any one of whole genome sequencing, CPN60 gene-based amplicon sequencing, other phylogenetically conserved genetic region-based amplicon sequencing, sequencing using approaches which involve either a fragment library or a mate-pair library or a paired-end library or a combination of the same. Further, the DNA extraction module 110 includes taxonomic classification of the sequenced reads at genus level using RDP, and latest version of any other taxonomic classification database such as Greengenes or Silva databases, or algorithms such as dada2 are covered in the scope of this invention

Further, at step 206 of the method 200, the quantitative abundance of each of a plurality of predetermined microbial marker sequences associated with the biological sample, is determined, from the extracted DNA sequences obtained at step 204 of the method 200. In an embodiment, the plurality of predetermined microbial marker sequences is associated to the microbes present in the biological sample. More specifically, the plurality of predetermined microbial marker sequences is associated to the microbes, and the corresponding operational taxonomy units (OTUs) are considered for obtaining their quantification from the biological sample.

Table 1 shows the plurality of predetermined microbial marker sequences associated with the stool sample. As shown in table 1, the plurality of predetermined microbial marker sequences associated with the stool sample are listed from Gut_seq1 to Gut_seq15 in the form of nucleic acid sequences.

Table 1

| Sequence ID | Microbial marker sequences for stool sample |
|---|---|
| Gut_seq1 | AGAGTTTGATCCTGGCTCAGGATGAACGCTAGCTACAGGC TTAACACATGCAAGTCGAGGGGCAGCATATTTCTAGCAAT AGAGATGATGGCGACCGGCGCACGGGTGAGTAACACGTAT CCAACCTGCCTTATACTCGGGGATAGCCTTTCGAAAGAAA GATTAATACCCGATGTTATAGTCTAACCGCATGATTTGACT |

(continued)

| Sequence ID | Microbial marker sequences for stool sample |
|---|---|
| | ATAAAAGATTTTCGGTATAAGATGGGGATGCGTTCCATTAG ATTGTAGGCGGGGTAACGGCCCACCTAGTCTTCGATGGAT AGGGGTTCTGAGAGGAAGGTCCCCCACATTGGAACTGAGA CACGGTCCAAACTCCTACGGGAGGCAGCAGTGAGGAATAT TGGTCAATGGGCGAGAGCCTGAACCAGCCAAGTAGCGTGA AGGATGACTGCCCTATGGGTTGTAAACTTCTTTTATATGGG AATAAAGTATTCCACGTGTGGGATTTTGTATGTACCATATG AATAAGGATCGGCTAACTCCGTGCCAGCAGCCGCGGTAAT ACGGAGGATCCGAGCGTTATCCGGATTTATTGGGTTTAAAG GGAGCGTAGGTGGACAGTTAAGTCAGTTGTGAAAGTTTGC GGCTCAACCGTAAAATTGCAGTTGATACTGGCTGTCTTGAG TACAGTAGAGGTGGGCGGAATTCGTGGTGTAGCGGTGAAA TGCTTAGATATCACGAAGAACTCCGATTGCGAAGGCAGCT CACTGGACTGCAACTGACACTGATGCTCGAAAGTGTGGGT ATCAAACAGGATTAGATACCCTGGTAGTCCACACAGTAAA CGATGAATACTCGCTGTTTGCGATATACAGTAAGCGGCCA AGCGAAAGCATTAAGTATTCCACCTGGGGAGTACGCCGGC AACGGTGAAACTCAAAGGAATTGACGGGGGCCCGCACAAG CGGAGGAACATGTGGTTTAATTCGATGATACGCGAGGAAC CTTACCCGGGCTTAAATTGCAAATGAATAATCTGGAAACA GGTTAGCCGCAAGGCATTTGTGAAGGTGCTGCATGGTTGTC GTCAGCTCGTGCCGTGAGGTGTCGGCTTAAGTGCCATAACG AGCGCAACCCTTATCTTTAGTTACTAACAGGTCATGCTGAG GACTCTAGAGAGACTGCCGTCGTAAGATGTGAGGAAGGTG GGGATGACGTCAAATCAGCACGGCCCTTACGTCCGGGGCT ACACACGTGTTACAATGGGGGGTACAGAAGGCCGCTACCT GGTGACAGGATGCTAATCCCTAAAACCTCTCTCAGTTCGGA TCGAAGTCTGCAACCCGACTTCGTGAAGCTGGATTCGCTAG |

(continued)

| Sequence ID | Microbial marker sequences for stool sample |
|---|---|
|  | TAATCGCGCATCAGCCATGGCGCGGTGAATACGTTCCCGG GCCTTGTACACACCGCCCGTCAAGCCATGAAAGCCGGGGG TACCTGAAGTACGTAACCGCAAGGAGCGTCCTAGGGTAAA ACTGGTAATTGGGGCTAAGTCGTAACAAGGTAACC |
| Gut_seq2 | GCGGCGAGCCTAATACACGCAAGTAGAAACGCTGAAGGGA GAGAGCTTCGCTCTTCTTCGGACGAGCTCGCGAAACGGGC GAGTAACGCGTAGGTAACCCGCCTCCGTAGCGGGGGATAA CTATTCGGAAACGATAGCTAATACCGCATAACAATAGGTG AACACATGTCATTTATTTGAAAGGGGCAATCGCTCCACTAC AAGATGGACCTGCGTTGTATTAGCTAGTAGGTGAGGTAAC GGCTCACCTAGGCGACGATACATAGCCGACCTGAGAGGGT GATCGGCCACACTGGGACTGAGACACGGCCCAGACTCCTA CGGGAGGCAGCAGTAGGGAATCTTCGGCAATGGGGGCAAC CCTGACCGAGCAACGCCGCGTGAGTGAAGAAGGTTTTCGG AACGTAAAGCTCTGTTGTAAGTCAAGAACGAGTGTGAGAG TGGAAAGTTCACACTGTGACGGTAGCTTACCAGAAAGGGA CGGCTAACTACGTGCCAGCAGCCGCGGTAATACGTAGGTC CCGAGCGTTGTCCGGATTTATTGGGCGTAAAGCGAGCGCA GGCGGTTTGATAAGTCTGAAGTTAAAGGCTGTGGCTCAAC CATAGTTCGCTTTGGAAACTGTCAAACTTGAGTGCAGAAG GGGAGAGTGGAATTCCATGTGTAGCGGTGAAATGCGTAGA TATATGGAGGAACACCGGTGGCGAAAGCGGCTCTCTGGTC TGTAACTGACGCTGAGGCTCGAAAGCGTGGGGAGCGAACA GGATTAGATACCCTGGTAGTCCACGCCGTAAACGATGAGT GCTAGGTGTTGGATCCTTTCCGGGATTCAGTGCCGCAGCTA ACGCATTAAGCACTCCGCCTGGGGAGTACGACCGCAAGGT TGAAACTCAAAGGAATTGACGGGGGCCCGCACAAGCGGTG GAGCATGTGGTTTAATTCGAAGCAACGCGAAGAACCTTAC |

(continued)

| Sequence ID | Microbial marker sequences for stool sample |
|---|---|
| | CAGGTCTTGACATCCCGATGCTATTTCTAGAGATAGAAAGT TACTTCGGTACATCGGTGACAGGTGGTGCATGGTTGTCGTC AGCTCGTGTCGTGAGATGTTGGGTTAAGTCCCGCAACGAG CGCAACCCCTATTGTTAGTTGCCATCATTCAGTTGGGCACT CTAGCGAGACTGCCGGTAATAAACCGGAGGAAGGTGGGGA TGACGTCAAATCATCATGCCCCTTATGACCTGGGCTACACA CGTGCTACAATGGTTGGTACAACGAGTTGCGAGTCGGTGA CGGCAAGCTAATCTCTTAAAGCCGAATCTCAGTTCGGATTG TAGGCTGCGAACTCGCCTACGTAAAGTCGGAAATCGCTAG GAATCGCGAATCAGCACGCCGCGGTAAAGACGTTCCCGGG CCTTGCCCCCACCGCCC |
| Gut_seq3 | CGCTGGCGGAACGCTTTACACATGCAAGTCGAACCTTTACG GGGGGAGGAGCTTGCTCCAGCCCAACAACGAGTGGCGAAC GGGTGAGGAATACATCGGAGCGTGACCGCTCGTGGGGGAC AACCAGCCGAAAGGTTGGCTAATACCGCATGAGTTCTACG GAAGAAAGAGGGGGACCCGCAAGGGCCTCTCGCGAGCGG AGCGGCCGATGACTGATTAGCCTGTTGGTGAGGTAACGGC TCACCAAAGCAACGATCAGTAGCTGGTCTGAGAGGACGAC CAGCCACACTGGGACTGAGACACGGACCAGACTCCTACGG GAGGCAGCAGTGGGGAATTTTGGACAATGGGCGCAAGCCT GATCCAGCTATTCCGCGTGTGGGATGAAGGCCCTCGGGTTG TAAACCACTTTTGTAGAGAACGAAAGACATCTTCGAATA AAGGATGTTGCTGACGGTACTCTAAGAATAAGCACCGGCT AACTACGTGCCAGCAGCCGCGGTAATACGTAGGGTGCGAG CGTTAATCGGAATTACTGGGCGTAAAGGGTGCGCAGGCGG TTGAGTAAGACAGATGTGAAATCCCCGAGCTTAACTCGGG AATGGCATATGTGACTGCTCGACTAGAGTGTGTCAGAGGG AGGTGGAATTCCACGTGTAGCAGTGAAATGCGTAGATATG |

(continued)

| Sequence ID | Microbial marker sequences for stool sample |
|---|---|
| | TGGAAGAACACCGATGGCGAAGGCAGCCTCCTGGGACATA ACTGACGCTCTGGCACGAAAGCGTGGGGAGCAAACAGGAT TAGATACTCTGGTAGTCCACGCCCTAAACGATGTTAACTAG TTGTTGGGATGTAATAATCTCAGTAACGCAGCCAACGCGA GAAGTTAACCGCCTGGGAAGTACGGTCGCAAGACTAAAAC TCAAAGGAATTGACGGGGACCCGCACAAGCGGTGGATGAT GTGGATTAATTCGATGCAACGCGAAAAACCTTACCTACCCT TGACATGTCAGGAAGCTCTTGTAATGAGAGCGTGTCCGCA AGGGAGCCTGAACACAGGTGGTGCATGGCTGTCGTCAGCT CGTGTCGTGAGATGTTGGGTTAAGTCCCGCAACGAGCGCA ATCCTTGTCACTAGTTGCTTCGAAAGGGCACTCTAGTGAGA CTGCCGGTGACAAACCGGAGGAAGGTGGGGATGACGTCAA GTCCTCATGGCCCTTATGGGTAGGGCTTCCCACGTCATACA ATGGTCGGAACAGAGGGCAGGGAAGCCGTGAGGCGGAGC CAATCCCAGAAAACCGATCGTAGTCCGGATTGCAGTTTGC AACTTGACTGCATGAAGTTGGAATCGCTAGTAATCGCGGA TCAGCATGCCGCGGTGAATACGTTCCCGGGTCTTGTACACC CCGCCCGTCAACCAATGGGAGTGGTGTTTCCCAGAAGTCGT TAGCCTAACCGCAAGGAGGGCGGCGACC |
| Gut_seq4 | GGCGTGCTTAACACATGCAAGTCGAGCGAAGCACTTAGGA AAGATTCTTCGGATGATTTCCTATTTGACTGAGCGGCGGAC GGGTGAGTAACGCGTGGGTAACCTGCCTCATACAGGGGGA TAACAGTTAGAAATGACTGCTAATACCGCATAAGACCACA GCACTGCATGGTGCAGGGGTAAAAACTCCGGTGGTATGAG ATGGACCCGCGTCTGATTAGTTAGTTGGTGGGGTAACGGCC TACCAAGGCGACGATCAGTAGCCGACCTGAGAGGGTGACC GGCCACATTGGGACTGAGACACGGCCCAAACTCCTACGGG AGGCAGCAGTGGGGAATATTGCACAATGGGGGAAACCCTG |

(continued)

| Sequence ID | Microbial marker sequences for stool sample |
|---|---|
| | ATGCAGCGACGCCGCGTGAGCGAAGAAGTATTTCGGTATG TAAAGCTCTATCAGCAGGGAAGAAAATGACGGTACCTGAC TAAGAAGCCCCGGCTAAATACGTGCCAGCAGCCGCGGTAA TACGTATGGAGCAAGCGTTATCCGGATTTACTGGGTGTAAA GGGAGTGTAGGTGGCCATGCAAGTCAGAAGTGAAAATCCG GGGCTCAACCCCGGAACTGCTTTTGAAACTGTAAGGCTAG AGTGCAGGAGGGGTGAGTGGAATTCCTAGTGTAGCGGTGA AATGCGTAGATATTAGGAGGAACACCAGTGGCGAAGGCGG CTCACTGGACTGTAACTGACACTGAGGCTCGAAAGCGTGG GGAGCAAACAGGATTAGATACCCTGGTAGTCCACGCCGTA AACGATGAATACTAGGTGTCGGGGCCCATAAGGGCTTCGG TGCCGCAGCAAACGCAATAAGTATTCCACCTGGGGAGTAC GTTCGCAAGAATGAAACTCAAAGGAATTGACGGGGACCCG CACAAGCGGTGGAGCATGTGGTTTAATTCGAAGCAACGCG AAGAACCTTACCAAGTCTTGACATCCCACTGACCGGACAG TAATGTGTCCTTTCCTTCGGGACAGTGGTGACAGGTGGTGC ATGGTTGTCGTCAGCTCGTGTCGTGAGATGTTGGGTTAAGT CCCGCAACGAGCGCAACCCCTATCCTTAGTAGCCAGCAGT AAGATGGGCACTCTAGGGAGACTGCCAGGGATAACCTGGA GGAAGGTGGGGATGACGTCAAATCATCATGCCCCTTATGA CTTGGGCTACACACGTGCTACAATGGCGTAAACAAAGTGA AGCGAAGTCGTGAGGCCAAGCAAATCACAAAAATAACGTC TCAGTTCGGATTGTAGTCTGCAACTCGACTACATGAAGCTG GAATCGCTAGTAATCGCAGATCAGAATGCTGCGGTGAATA CGTTCCCGGGTCTTGTACACACCGCCCGTCACACCATGGGA GTCGAAAATGCCCGAAGTCGGTGACCTAACGAAAGAAGGA GCCGCCGAAGGCAGGTTTGATAACTGGGGTGAA |

(continued)

| Sequence ID | Microbial marker sequences for stool sample |
| --- | --- |
| Gut_seq5 | GACGAACTCTGGCGGCGCGCCTAACACATGCAAGTCGAAC GGAGCTTAGAGAGCTTGCTTTTTAAGCTTAGTGGCGAACGG GTGAGTAACGCGTGGATAATCTACCCTTAAGATGGGGATA ACGGCTGGAAACGGTCGCTAATACCGAATACGCTCCCGAT TTTATCGGTGGGGGGAAAGATGGCCTCTGCTTGCAAGCTAT CGCTTAAGGATGAGTCCGCGTCCCATTAGCTAGTTGGCGGG GTAACGGCCCACCAAGGCGACGATGGGTAGCCGGTCTGAG AGGATGACCGGCCACACTGGAACTGGAACACGGTCCAGAC TCCTACGGGAGGCAGCAGTGGGGAATATTGCGCAATGGGC GAAAGCCTGACGCAGCGACGCCGCGTGAGGGATGAAGGTT CTCGGATCGTAAACCTCTGTCAGGGGGGAAGAAACCCCCT CGTGTGAATAATGCGAGGGCTTGACGGTACCCCCAAAGGA AGCACCGGCTAACTCCGTGCCAGCAGCCGCGGTAATACGG AGGGTGCAAGCGTTAATCGGAATCACTGGGCGTAAAGCGC ACGTAGGCGGCTTGGTAAGTCAGGGGTGAAATCCCACAGC CCAACTGTGGAACTGCCTTTGATACTGCCAGGCTTGAGTAC CGGAGAGGGTGGCGGAATTCCAGGTGTAGGAGTGAAATCC GTAGATATCTGGAGGAACACCGGTGGCGAAGGCGGCCACC TGGACGGTAACTGACGCTGAGGTGCGAAAGCGTGGGTAGC AAACAGGATTAGATACCCTGGTAGTCCACGCTGTAAACGA TGGGTGCTGGGTGCTGGGATGTATGTCTCGGTGCCGTAGCT AACGCGATAAGCACCCCGCCTGGGGAGTACGGTCGCAAGG CTGAAACTCAAAGAAATTGACGGGGGCCCGCACAAGCGGT GGAGTATGTGGTTTAATTCGATGCAACGCGAAGAACCTTA CCCAGGCTTGACATCTAGGGAACCCTTCGGAAATGAAGGG GTGCCCTTCGGGGAGCCCTAAGACAGGTGCTGCATGGCTG TCGTCAGCTCGTGCCGTGAGGTGTTGGGTTAAGTCCCGCAA CGAGCGCAACCCCTATCTTCAGTTGCCAGCAGGTAAGGCT |

(continued)

| Sequence ID | Microbial marker sequences for stool sample |
|---|---|
| | GGGCACTCTGGAGAGACCGCCCCGGTCAACGGGGAGGAAG GTGGGGACGACGTCAAGTCATCATGGCCCTTACGCCTGGG GCTACACACGTACTACAATGGCGCGCACAAAGGGTAGCGA GACCGCGAGGTGGAGCCAATCCCAAAAAACGCGTCCCAGT CCGGATTGGAGTCTGCAACTCGACTCCATGAAGTCGGAAT CGCTAGTAATTCGAGATCAGCATGCTCGGGTGAATGCGTTC CCGGGCCTTGCAC |
| Gut_seq6 | GAGTGGCGGACGGGTGAGTAACGCGTGGGTAACCTGCCTC ATACAGGGGGATAACAGTTAGAAATGACTGCTAATACCGC ATAAGCGCACAGTACCGCATGGTACGGTGTGAAAAACTCC GGTGGTATGAGATGGACCCGCGTCTGATTAGCTAGTTGGC GGGGTAACGGCCCACCAAGGCGACGATCAGTAGCCGACCT GAGAGGGTGACCGGCCACATTGGGACTGAGACACGGCCCA AACTCCTACGGGAGGCAGCAGTGGGGAATATTGCACAATG GGCGAAAGCCTGATGCAGCGACGCCGCGTGAACGAAGAA GTATTTCGGTATGTAAAGTTCTATCAGCAGGGAAGATAATG ACGGTACCTGACTAAGAAGCACCGGCTAAATACGTGCCAG CAGCCGCGGTAATACGTATGGTGCAAGCGTTATCCGGATTT ACTGGGTGTAAAGGGAGCGCAGGCGGTACGGCAAGTCTGA TGTGAAAGCCCGGGGCTCAACCCCGGTACTGCATTGGAAA CTGTCGAACTAGAGTGTCGGAGGGGTAAGCGGAATTCCTA GTGTAGCGGTGAAATGCGTAGATATTAGGAGGAACACCAG TGGCGAAGGCGGCTTACTGGACGATAACTGACGCTGAGGC TCGAAAGCGTGGGGAGCAAACAGGATTAGATACCCTGGTA GTCCACGCCGTAAACGATGAATACTAGGTGTCGGGTCCCA CAGGGATTCGGTGCCGCAGCAAACGCAATAAGTATTCCAC CTGGGGAGTACGTTCGCAAGAATGAAACTCAAAGGAATTG ACGGGGACCCGCACAAGCGGTGGAGCATGTGGTTTAATTC |

(continued)

| Sequence ID | Microbial marker sequences for stool sample |
|---|---|
| | GAAGCAACGCGAAGAACCTTACCAAGTCTTGACATCCCGA TGACCGGTACTTAACCGTACCTTTTCTTCGGAACATCGGTG ACAGGTGGTGCATGGTTGTCGTCAGCTCGTGTCGTGAGATG TTGGGTTAAGTCCCGCAACGAGCGCAACCCCTGTTCTTAGT AGCCAGCGGTTTGGCCGGGCACTCTAGGAAGACTGCCAGG GATAACCTGGAGGAAGGCGGGGATGACGTCAAATCATCAT GCCCCTTATGACTTGGGCTACACACGTGCTACAATGGCGTA AACAAAGGGAAGCAAAGCTGTGAGGCCGAGCAAATCTCA AAAATAACGTCTCAGTTCGGATTGTAGTCTGCAACTCGACT ACATGAAGCTGGAATCGCTAGTAATCGCGAATCAGAATGT CGCGGTGAATACGTTCCCGGGTCTTGTACACACCGCCCGTC ACACCATGGGAGTTGGAAATGCCCGAAGTCAGTGACCCAA CCGCAAGGAGGGAGCTGCCGA |
| Gut_seq7 | ATGAACGCTGGCGGCGTGCCTAACACATGCAAGTCGAGCG AAGCACTTGCAAATGATCCTTCGGGTGATTTTGCTGGTGAC TGAGCGGCGGACGGGTGAGTAACGCGTGGGTAACCTGCCT CATACAGGGGGATAACAGTTAGAAATGACTGCTAATACCG CATAAGCGCACAGTACCACATGGTACGGTGTGAAAAACTC CGGTGGTATGAGATGGACCCGCGTCTGATTAGCTAGTTGGC GGGGTAACGGCCCACCAAGGCGACGATCAGTAGCCGACCT GAGAGGGTGATCGGCCACATTGGGACTGAGACACGGCCCA AACTCCTACGGGAGGCAGCAGTGGGGAATATTGCACAATG GGGGAAACCCTGATGCAGCAACGCCGCGTGAGTGAAGAAG TATTTCGGTATGTAAAGCTCTATCAGCAGGGAAGATAATG ACGGTACCTGACTAAGAAGCCCCGGCTAACTACGTGCCAG CAGCCGCGGTAATACGTAGGGGGCAAGCGTTATCCGGATT TACTGGGTGTAAAGGGAGCGTAGACGGCGCAGCAAGTCTG ATGTGAAAGGCAGGGGCTTAACCCCTGGACTGCATTGGAA |

(continued)

| Sequence ID | Microbial marker sequences for stool sample |
|---|---|
| | ACTGCTGTGCTTGAGTGCCGGAGGGGTAAGCGGAATTCCT AGTGTAGCGGTGAAATGCGTAGATATTAGGAGGAACACCA GTGGCGAAGGCGGCTTACTGGACGGTAACTGACGTTGAGG CTCGAAAGCGTGGGGGAGCAAACAGGATTTAGATACCCTG GTAGTCCACGCCGTAAACGATGAATACTAGGTGTCAGGGA GCACAGCTCCTTTTGGTGCCGCCGCAAAGGCATTAAGTATT CCACCGGGGGAGTACGTTCGCAAGAATGAAACTCAAAGGA ATTGACGGGGACCCGCACAAGCGGTGGAGCATGTGGTTTA ATTCGAAGCAACGCGAAGAACCTTACCAAATCTTGACATC CCTCTGACCGGGACTTAACCGTCCCTTTCTTTCGGGACAGG GGAGACAGGTGGTGCATGGTTGTCGTCAGCTCGTGTCGTG AGATGTTGGGTTAAGTCCCGCAACGAGCGCAACCCCTATC CTTAGTAGCCAGCACGCAGTGGTGGGCACTCTGAGGAGAC TGCCAGGGATAACCTGGAGGAAGGCGGGGATGACGTCAAA TCATCATGCCCCTTATGATTTGGGCTACACACGTGCTACAA TGGCGTAAACAAAGGGAAGCGAACCCGCGAGGGTGGGCA AATCTCAAAAATAACGTCCCAGTTCGGACTGCAGTCTGCA ACTCGACTGCAGGAAGCTGGAATCGCTAGTAATCGCGGAT CAGAATGCCGCGGTGAATACGTTCCCGGGTCTTGTACACAC CGCCCGTCACACCATGGGAGTCAGTAACGCCCGAAGTCAG TGACCTAACCGCAAGGGAGGAGCTGCCGAAGGCGGGACCG ATGA |
| Gut_seq8 | CTCAGGATGAACGCTAGCTACAGGCTTAACACATGCAAGT CGAGGGGCAGCATGGTCTTAGCTTGCTAAGGCTGATGGCG ACCGGCGCACGGGTGAGTAACACGTATCCAACCTGCCGTC TACTCTTGGCCAGCCTTCTGAAAGGAAGATTAATCCAGGAT GGGATCATGAGTTCACATGTCCGCATGATTAAAGGTATTTT CCGGTAGACGATGGGGATGCGTTCCATTAGATAGTAGGCG |

(continued)

| Sequence ID | Microbial marker sequences for stool sample |
|---|---|
|  | GGGTAACGGCCCACCTAGTCAACGATGGATAGGGGTTCTG AGAGGAAGGTCCCCCACATTGGAACTGAGACACGGTCCAA ACTCCTACGGGAGGCAGCAGTGAGGAATATTGGTCAATGG GCGATGGCCTGAACCAGCCAAGTAGCGTGAAGGATGACTG CCCTATGGGTTGTAAACTTCTTTTATAAAGGAATAAAGTCG GGTATGCATACCCGTTTGCATGTACTTTATGAATAAGGATC GGCTAACTCCGTGCCAGCAGCCGCGGTAATACGGAGGATC CGAGCGTTATCCGGATTTATTGGGTTTAAAGGGAGCGTAG ATGGATGTTTAAGTCAGTTGTGAAAGTTTGCGGCTCAACCG TAAAATTGCAGTTGATACTGGATGTCTTGAGTGCAGTTGAG GCAGGCGGAATTCGTGGTGTAGCGGTGAAATGCTTAGATA TCACGAAGAACTCCGATTGCGAAGGCAGCCTGCTAAGCTG CAACTGACATTGAGGCTCGAAAGTGTGGGTATCAAACAGG ATTAGATACCCTGGTAGTCCACACGGTAAACGATGAATAC TCGCTGTTTGCGATATACTGCAAGCGGCCAAGCGAAAGCG TTAAGTATTCCACCTGGGGAGTACGCCGGCAACGGTGAAA CTCAAAGGAATTGACGGGGGCCCGCACAAGCGGAGGAAC ATGTGGTTTAATTCGATGATACGCGAGGAACCTTACCCGGG CTTAAATTGCAGATGAATTACGGTGAAAGCTGTAAGCCGC AAGGCATCTGTGAAGGTGCTGCATGGTTGTCGTCAGCTCGT GCCGTGAGGTGTCGGCTTAAGTGCCATAACGAGCGCAACC CTTGTTGTCAGTTACTAACAGGTTTTGCTGAGGATTCTGAC AAGACTGCCATCGTAAGATGTGAGGAAGGTGGGGATGACG TCAAATCAGCACGGCCCTTATGTCCGGGGCTACACACGTGT TACAATGGGGGGTACAGAGGGCTGCTACCACGCAAGTGGA TGCCAATCCCAAAAACCTCTGTCAGTTCGGATTGAAGTCTG CAACCCGACTTCATGAAGCTGGATTCGCTAGTAATCGCGCA TCAGCCACGGCGCGGTGAATACGTTCCCGGGCCTTGTACAC TCCGCCCGTCAATCCATGGGAGCCGGGGGTACCTGAAGTG CGTAACCGCAAGGAG |

(continued)

| Sequence ID | Microbial marker sequences for stool sample |
|---|---|
| Gut_seq9 | GGATGAACGCTGGCGGCGTGCTTAACACATGCAAGTCGAA CGAAGCAATCTAGCGGAAGTTTTCGGATGGAAGCTGGATT GACTGAGTGGCGGACGGGTGAGTAACGCGTGGGTAACCTG CCTCACACTGGGGGACAACAGTTAGAAATGACTGCTAATA CCGCATAAGCGCACAGGACCGCATGGTCCGGTGTGAAAAA CTCCGGTGGTGTGAGATGGACCCGCGTTTGATTAGCTAGTT GGTGGGGTAACGGCCTACCAAGGCGACGATCAATAGCCGA CCTGAGAGGGTGACCGGCCACATTGGGACTGAGACACGGC CCAAACTCCTACGGGAGGCAGCAGTGGGGAATATTGCACA ATGGGGGAAACCCTGATGCAGCGACGCCGCGTGAGTGAAG AAGTATTTCGGTATGTAAAGCTCTATCAGCAGGGAAGAAA ATGACGGTACCTGACTAAGAAGCCCCGGCTAACTACGTGC CAGCAGCCGCGGTAATACGTAGGGGGCAAGCGTTATCCGG ATTTACTGGGTGTAAAGGGAGCGTAGACGGTAAAGCAAGT CTGAAGTGAAAGCCCGGGGCTCAACCCCGGGACTGCTTTG GAAACTGTTTAACTAGAGTGCTGGAGAGGTAAGCGGAATT CCTAGTGTAGCGGTGAAAATGCGTAGATATTACGAGGAAC ACCCATTGGCGAAGGGGGCTTATTGGACAAGTAACTGACG TTGAGGCTTGAAAAGCGTGGGGAGCAAACAGGATTAGATA CCCTGGTAGTCCACCCCGTAAACGATGAATATTAGGTGTGT GGGGACAAAGTCGTTCGGTGCCGTCGCAAACGCAATAAGT ATTCCACGTGGGGAGTACGTTCGCAAGAAAGAAACTCAAA GGAATTGACGCGGACCCGCACAAGCGGTGGAGCACGTGGT TTAATTTGAAGCAACGCGAAGAACCTTACCAAATCTTGAC ATCCTCTGACCGGCGAGTAATGTCTCCTTTCTTTCGGGAC AGAGGAGACAGGTGGTGCATGGTTGTCGTCAGCTCGTGTC |

(continued)

| Sequence ID | Microbial marker sequences for stool sample |
|---|---|
|  | GTGAGATGTTGGGTTAAGTCCCGCAACGAGCGCAACCCTT ATCTTAAGTAGCCAGCAGTTCGGCTGGGCACTCTTGGGAG ACTGCCAGGGATAACCTGGAGGAAGGTGGGGATGACGTCA AATCATCATGCCCCTTATGATTTGGGCTACACACGTGCTAC AATGGCGTAAACAAAGAGAGGCGAGACCGCGAGGTGGAG CAAATCTCAAAAATAACGTCTCAGTTCGGACTGCAGGCTG CAACTCGCCTGCACGAAGCTGGAATCGCTAGTAATCGCGA ATCAGAATGTCGCGGTGAATACGTTCCCGGGTCTTGTACAC ACCGCCCGTCACACCATGGGAGTCAGTAATGCCCGAAGTC AGTGACCCAACCGAAAGAGGGAGCTGCCGAAGGCAGGA CCGATAACTG |
| Gut_seq1 0 | AACACATGCAAGTCGAGCGATGAAATTTTCCCTGAACCCTT CGGGGTGAAGACAAAATGGATTAGCGGCGGACGGGTGAGT AACACGTGAGTAACCTGCCTTAGACATTGTGATAGCCTCGG GAAACCGGGATTAATACCGAATAAAATCATAGGTGCACAT GCACTAATGATCAAAACTCCGGTGGTCTAAGATGGACTCG CGTCCCATTAGCTAGTTGGTAGGGTAACGGCCTACCAAGG CGACGATGGGTAGCCGGCCTGAGAGGGTGAACGGCCACAT TGGAACTGAGAAACGGTCCAAACTCCTACGGGAGGCAGCA GTGGGGAATATTGCACAATGGAGGGAACTCTGATGCAGCG ACGCCGCGTGAACGAAGAAGGCTTTCGAGTCGTAAAGTTC TTTTATATGGGAAGATAATGACGGTACCATAAGAAAAAGC TCCGGCTAACTACGTGCCAGCAGCCGCGGTAATACGTAGG GGGCTAGCGTTGTCCGGAATCACTGGGCGTAAAGGGTTCG CAGGCGGAAATGCAAGTCAGGTGTAAAAGGCAGTAGCTTA ACTACTGTAAGCATTTGAAACTGCATATCTTGAAGAGTA GAGGTAAGTGGAATTTTTAGTGTAGCGGTGAAATGCGTAG ATATTAAAAAGAATACCGGTGGCGAAGGCGACTTACTGGG |

(continued)

| Sequence ID | Microbial marker sequences for stool sample |
|---|---|
| | CTCATTCTGACGCTGAGGAACGAAAGCGTGGGTAGCAAAC AGGATTAGATACCCTGGTAGTCCACGCTGTAAACGATGAG TGCTAGGTGTCGGCGTAAGTCGGTGCCGCAGTTAACACAA TAAGCACTCCGCCTGGGGAGTACGTGCGCAAGCATGAAAC TCAAAGGAATTGACGGGGACCCGCACAAGCAGCGGAGCAT GTGGTTTAATTCGAAGCAACGCGAAGAACCTTACCAGGGC TTGACATACTGAGGACGTATTTAGAGATAAATATTCCTCTT CGGAGGCCTCAATACAGGTGGTGCATGGTTGTCGTCAGCTC GTGTCGTGAGATGTTGGGTTAAGTCCCGCAACGAGCGCAA CCCTTATCTTTAGTTGCCAGCATTTCGGATGGGAACTCTAA AGAGACTGCCGATGACAAATCGGAGGAAGGTGGGGATGA CGTCAAATCATCATGCCCTATATGCCCTGGGTTACACACGT GCTACAATGGAAGGTACAAAGGGAAGCAAGATAGTGATAT TAAGCAAACCTCAAAAAGCCTTTCCCAGTTCGGATTGTACT CTGCAACTCGAGTACATGAAGATGGAGTTGCTAGTAATCG CAGATCAGAATGCTGTGGTGAATGCGTTCCCGGGTCTTGTA CACACCGCCCGTCACACCATGGGAGCCGGGGGTACCTGAA GTGCGTAACCGCGAGGAGCGCCCTAGGGTAAAACTGGTGA CTGGGGCTAA |
| Gut_seq1 1 | GCTGGCGGCGCGCCTAACACATGCAAGTCGCCCTTGCGAG AGAGAGCTTGCTTTCTCGAGCGAGTGGCGAACGGGTGAGT AACGCGTGAGGAACCTGCCTCAAAGAGGGGGACAACAGTT GGAAACGACTGCTAATACCGCATAAGCCCACGACCCGGCA TCGGGTAGAGGGAAAAGGAGCAATCCGCTTTGAGATGGCC TCGCGTCCGATTAGCTAGTTGGTGAGGTAACGGCCCACCA AGGCGACGATCGGTAGCCGGACTGAGAGGTTGAACGGCCA CATTGGGACTGAGACACGGCCCAGACTCCTACGGGAGGCA GCAGTGGGGAATATTGCACAATGGGGGAAACCCTGATGCA |

(continued)

| Sequence ID | Microbial marker sequences for stool sample |
|---|---|
| | GCGACGCCGCGTGGAGGAAGAAGGTCTTCGGATTGTAAAC TCCTGTTGTTGAGGAAGATAATGACGGTACTCAACAAGGA AGTGACGGCTAACTACGTGCCAGCAGCCGCGGTAAAACGT AGGTCACAAGCGTTGTCCGGAATTACTGGGTGTAAAGGGA GCGCAGGCGGGAAGACAAGTTGGAAGTGAAATCCATGGGC TCAACCCATGAACTGCTTTCAAAACTGTTTTTCTTGAGTAG TGCAGAGGTAGGCGGAATTCCCGGTGTAGCGGTGGAATGC GTAGATATCGGGAGGAACACCAGTGGCGAAGGCGGCCTAC TGGGCACCAACTGACGCTGAGGCTCGAAAGTGTGGGTAGC AAACAGGATTAGATACCCTGGTAGTCCACACTGTAAACGA TGATTACTAGGTGTTGGAGGATTGACCCTTCCAGCGCCGCA GTAATCACAATAAGTAATCCACATGGCGAGTACGACCGCA AGGTTGAGACTCAAAGGAATTGACGGGGGCCCGCACAAGC AGTGGAGTATGTGGTGTAATTCGACGCAACGCGAAGAACC TTAGCAAGTCTTGACATCCTGCGACGGTTCTGGAAACAGA ACTTTCCTTCGGGACGCAGAGACAGGTGGTGCATGGTTGTC GTCAGCTCGTGTCGTGAGATGTTGGGTTAAGTCCCGCAACG AGCGCAACCCTTATGGTCAGTTACTACGCAAGAGGACTCT GGCCAGACTGCCGTAGACAAAACGGAGGAAGGTGGGGAT GACGTCAAATCATCATGCCCTTTATGACTTGGGTTACACAC GTACTACAATGGCGTTAAACAAAGAGAAGCAAGACCGCGA GGTGGAGCAAAACTCAGAAACATCGTCTCAGTTTGGAATG CAGGCTGCAACTCGCCTGCACGAAGTCGGAATTGCTAGTA ATCGCGGATCAGCATGGTGCGGTGAATACGTTCCCGGGCC TTGTACACACCGCCCGTCACACCATGAGAGCCGGGGGGAC CCGAAGTCGGTAGTCTAACTGTAAGGAGGACGCCGCCGAA GGTAAAACT |

(continued)

| Sequence ID | Microbial marker sequences for stool sample |
|---|---|
| Gut_seq1 2 | GACGAACGCTGTCGGCGCGCTTAACACATGCAAGTCGAGC GATGAAATTTTGACAGATCCCTTCGGGGTGAAGATAAAAT GGATTAGCGGCGGACGGGTGAGTAACGCGTGGGTAACCTG CCCCATACAGGGGGATAACAGTTAGAAATGACTGCTAATA CCGCATAAGACCACAGCGCCGCATGGTGCAGGGGTAAAAA CTCCGGTGGTATGGGATGGACCCGCGTCTGATTAGCTAGTT GGCGGGGTAACGGCCCACCAAGGCGACGATCAGTAGCCGA CCTGAGAGGGTGACCGGCCACATTGGGACTGAGACACGGC CCAAACTCCTACGGGAGGCAGCAGTGGGGAATATTGCACA ATGGGGGAAACCCTGATGCAGCGACGCCGCGTGAGTGATG AAGTATTTCGGTATGTAAAGCTCTATCAGCAGGGAAGAAA ATGACGGTACCTGACTAAGAAGCCCCGGCTAACTACGTGC CAGCAGCCGCGGTAATACGTAGGGGGCAAGCGTTATCCGG ATTTACTGGGTGTAAAGGGAGCGTAGACGGCTGTGCAAGT CTGGAGTGAAAGCCCGGGGCTCAACCCCGGGACTGCTTTG GAAACTGTACGGCTGGAGTGCTGGAGAGGCAAGCGGAATT CCTAGTGTAGCGGTGAAATGCGTAGATATTAGGAGGAACA CCAGTGGCGAAGGCGGCTTGCTGGACAGTAACTGACGTTG AGGCTCGAAAGCGTGGGGAGCAAACAGGATTAGATACCCT GGTAGTCCACGCCGTAAACGATGAATGCTAGGTGTCGGGG AGCAAAGCTCTTCGGTGCCGCCGCAAACGCAATAAGCATT CCACCTGGGGAGTACGTTCGCAAGAATGAAACTCAAAGGA ATTGACGGGGACCCGCACAAGCGGTGGAGCATGTGGTTTA ATTCGAAGCAACGCGAAGAACCTTACCAAGTCTTGACATC CCCCTGACCGGCAAGTAATGTTGCCTTTCCTTCGGGACAGG GGAGACAGGTGGTGCATGGTTGTCGTCAGCTCGTGTCGTG AGATGTTGGGTTAAGTCCCGCAACGAGCGCGACCCTTATCC TCAGTAGCCAGCAGGTGAAGCTGGGCACTCTGTGGAGACT |

(continued)

| Sequence ID | Microbial marker sequences for stool sample |
|---|---|
| | GCCAGGGATAACCTGGAGGAAGGTGGGGACGACGTCAAAT CATCATGCCCCTTATGACTTGGGCTACACACGTGCTACAAT GGCGTAAACAAAGGGAAGCGAGAGGGTGACCTGGAGCAA ATCCCAAAAATAACGTCTCAGTTCGGATTGTAGTCTGCAAC TCGACTACATGAAGCTGGAATCGCTAGTAATCGCGAATCA GCATGTCGCGGTGAATACGTTCCCGGGTCTTGTACACACCG CCCGTCACACCATGGGAGTCAGCAACGCCCGAAGCCGGTG ACCTAACCGCAAGGAAGGAGCCGTCGAAGGCGGGGCAGG TAACTGGGGTGAA |
| Gut_seq1 3 | GCTTAACACATGCAAGTCGGGGGGCAGCATTCCTTTTTGCT TGCAAACTGGAGATGGCGACCGGCGCACGGGTGAGTAACA CGTATCCAACCTGCCGATAACTCGGGGATAGCCTTTCGAAA GAAAGATTAATACCCGATGGTATAATCAGACCGCATGGTC TTGTTATTAAAGAATTTCGGTTATCGATGGGGATGCGTTCC ATTAGGCAGTTGGTGAGGTAACGGCTCACCAAACCTTCGA TGGATAGGGGTTCTGAGAGGAAGGTCCCCCACATTGGAAC TGAGACACGGTCCAAACTCCTACGGGAGGCAGCAGTGAGG AATATTGGTCAATGGGCGCAGGCCTGAACCAGCCAAGTAG CGTGAAGGATGACTGCCCTATGGGTTGTAAACTTCTTTTAT ATGGGAATAAAGTTTTCCACGTGTGGAATTTTGTATGTACC ATATGAATAAGGATCGGCTAACTCCGTGCCAGCAGCCGCG GTAATACGGAGGATCCGAGCGTTATCCGGATTTATTGGGTT TAAAGGGAGCGTACGTGGACAGTTAAGTCAGTTGTGAAAG TTTGCGGCTCAACCGTAAAATTGCAGTTGATACTGGCTGTC TTGAGTACAGTAGAGGTGGGCGGAATTCGTGGTGTAGCGG TGAAATGCTTAGATATCACGAAGAACTCCGATTGCGAAGG CAGCTCACTGGACTGCAACTGACACTGATGCTCGAAAGTG TGGGTATCAAACAGGATTAGATACCCTGGTAGTCCACACA |

(continued)

| Sequence ID | Microbial marker sequences for stool sample |
|---|---|
| | GTAAACGATGAATACTCGCTGTTTGCGATATACAGTAAGC GGCCAAGCGAAAGCATTAAGTATTCCACCTGGGGAGTACG CCGGCAACGGTGAAACTCAAAGGAATTGACGGGGGCGCGC ACAAGCGGAGGAACATGTGGTTTAATTAGATGATACGCGA GGAACCTTACCCGGGCTTAAATTGCATTTGAATATATTGGT AACAGTATAGTCGTAAGACAAATGTGAAGGTGCTGCATGG TTGTCGTCAGCTCGTGCCGTGAGGTGTCGGCTTAAGTGCCA TAACGAGCGCAACCCTTATCTTTAGTTATTAACAGGTCATG CTGAGGACTCTAGAGAGACTGCCGTGGTAAGATGTGAGGA AGGTGGGGATGACGTCAAATCAGCACGGCCCTTACGTCCG GGGCTACACGTGTTACAATGGGGGGGTACAGAAGGCAGG TACCTGGTGACAGGATGGTAATCCCAAAAGCCTTTGTCAGT TAGGATGGAAGTCTGCAACCCGAATTCGTGAAGCTGGATT AGCTAGTAATCGCGCATCAGCCAGGGTGCGGTGAATACGT TCCCGGGCCTTGTACACCCCGCCCGTCAAGCCATGAAAGCC GGGGGTACGTGAAGTACGTAAAAACAAGGAGCGTCCTAGG GTAAAACTGGTAAT |
| Gut_seq1 4 | AGAGTTTGATCCTGGCTCAGGATGAACGCTGGCGGCGTGC CTAACACATGCAAGTCGAACGGAGTCGTTTTGGAAAATCC TTCGGGATTGGAATTCTCGACTTAGTGGCGGACGGGTGAGT AACGCGTGAGCAATCTGCCTTTAAGAGGGGGATAACAGTC GGAAACGGCTGCTAATACCGCATAAAGCATTAAATTCGCA TGTTTTTGATGCCAAAGGAGCAATCCGCTTCTAGATGAGCT CGCGTCTGATTAGCTGGTTGGCGGGGTAACGGCCCACCAA GGCGACGATCAGTAGCCGGACTGAGAGGTTGAACGGCCAC ATTGGGACTGAGACACGGCCCAGACTCCTACGGGAGGCAG CAGTGGGGAATATTGCGCAATGGGGGAAGCCCTGACGCAG CAACGCCGCGTGATTGAAGAAGGCCTTCGGGTTGTAAAGA |

(continued)

| Sequence ID | Microbial marker sequences for stool sample |
|---|---|
| | TCTTTAATCAGGGACGAAACAAATGACGGTACCTGAAGAA TAAGCTCCGGCTAACTACGTGCCAGCAGCCGCGGTAATAC GTAGGGAGCAAGCGTTATCCGGATTTACTGGGTGTAAAGG GCGCGCAGGCGGGCCGGCAAGTTGGAAGTGAAATCTATGG GCTTAACCCATAAACTGCTTTCAAAACTGCTGGTCTTGAGT GATGGAGAGGCAGGCGGAATTCCGTGTGTAGCGGTGAAAT GCGTAGATATACGGAGGAACACCAGTGGCGAAGGCGGCTT GCTGGACATTAACTGACGCTGAGGCGCGAAAGCGTGGGGA GCAAACAGGATTAGATACCCTGGTAGTCCACGCCGTAAAC GATGGATACTAGGTGTGGGAGGTATTGACCCCTTCCGTGCC GCAGTTAACACAATAAGTATCCCACCTGGGGAGTACGGCC GCAAGGTTGAAACTCAAAGGAATTGACGGGGGCCCGCGCA AGCAGTGGAGTATGTGGTTTAATTCGAAGCAACGCGAAGA ACCTTACCAGGTCTTGACATCCCGATGACCGCCTTAGAGAT AAGGCTTTTCTTCGGAACATCGGTGACAGGTGGTGCATGGT TGTCGTCAGCTCGTGTCGTGAGATGTTGGGTTAAGTCCCGC AACGAGCGCAACCCTTACGGTTAGTTGATACGCAAGATCA CTCTAGCCGGACTGCCGTTGACAAAACGGAGGAAGGTGGG GACGACGTCAAATCATCATGCCCCTTATGACCTGGGCTACA CACGTACTACAATGGCAGTCATACAGAGGGAAGCAAAACC GCGAGGTGGAGCAAATCCCTAAAAGCTGTCCCAGTTCAGA TTGCAGGCTGCAACCCGCCTGCATGAAGTCGGAATTGCTA GTAATCGCGGATCAGCATGCCGCGGTGAATACGTTCCCGG GCCTTGTACACACCGCCCGTCACACCATGAGAGCCGTCAAT ACCCGAAGTCCGTAGCCTAACCGTAAGGAGGGCGCGGCCG AAGGTAGGGGTGGTAATTAGGGTGAAGTCGTAACAAGGTA GCCGTATCGGAAGGTGCGGCTGGATCACCTCCTT |

(continued)

| Sequence ID | Microbial marker sequences for stool sample |
|---|---|
| Gut_seq1 5 | CTTACACATGCAGTCGAACGGGAAGAGATGAAGAGCTTGC<br>TCTTTATCGAATCCAGTGGCAAACGGGTGAGTAACACGTA<br>AACGACCTGCCTTCAGGATGGGGACAACAGACGGAAACGA<br>CTGCTAATACCGAATACGTTCCACGGGCCGCATGACCTGTG<br>GAAGAAAGGGTAGCCTCTACCTGTAAGCTATCGCCTGAAG<br>AGGGGTTTGCGTCTGATTAGGCAGTTGGTGGGGTAACGGC<br>CCACCAAACCAACGATCAGTAGCCGGTCTGAGAGGATGAA<br>CGGCCACACTGGAACTGAGACACGGTCCAGACTCCTACGG<br>GAGGCAGCAGTGGGGAATCTTCCGCAATGGACGAAAGTCT<br>GACGGAGCAACGCCGCGTGAGTGAAGACGGCCTTCGGGTT<br>GTAAAGCTCTGTGATCCGGGACGAAAGAGCCTGAGGTGAA<br>TAGCCTAAGGAAGTGACGGTACCGGAAAAGCAAGCCACGG<br>CTAACTACGTGCCAGCAGCCGCGGTAATACGTAGGTGGCA<br>AGCGTTGTCCGGAATTATTGGGCGTAAAGCGCGCGCAGGC<br>GGCTTCCTAAGTCCATCTTAAAAGTGCGGGGCTTAACCCCG<br>TGATGGGATGGAAACTGGGAAGCTGGAGTATCGGAGAGGA<br>AAGGTGGAATTCCTAGTGTAGCGGTGAAATGCGTAGAGAT<br>TAGGAAGAACACCGGTGGCGAAGGCGACTTTCTGGACGAA<br>AACTGACGCTGAGGCGCGAAAGCGTGGGGAGCAAACAGG<br>ATTAGATACCCTGGTAGTCCACGCCGTAAACGATGGATAG<br>GGGGGGGGGTGGGGGCTCCCCCCTTCTGTGCCGGAGTTAA<br>CGCAATAAGTATCCCGCCTGGGAAGTACGATCGCAAGATT<br>AAAACTCAAAGGAATTGACGGGGGCCCGCACAAGCGGTGG<br>AGTATGTGGTTTAATTCGACGCAACGCGAAGAACCTTACC<br>AGGTCTTGACATTGATCGCAATTTTCAGAAATGAGAAGTTC<br>TCCTTCGGGAGACGAGAAACAGGTGGTGCACGGCTGTCG<br>TCAGCTCGTGTCGTGAGATGTTGGGTTAAGTCCCGCAACGA<br>GCGCAACCCCTATCATTTGTTGCCAGCACGTAAAGGTGGG |

(continued)

| Sequence ID | Microbial marker sequences for stool sample |
|---|---|
| | GACTCAAATGAGACCGCCGCAGACAATGCGGAGGAAAGGT GGGGATGACGTCAAGTCATCATGCCCCTTATGACCTGGGCT ACACACGTACTACAATGGGTGTCAACAAAGAGAAGCGAAA GGGCGACCTGGAGCCAACCTCAAAAACACACCCCCAGTTC AGATCGCAGGCTGCAACTCGCCTGCGTGAAGCAGGAATCG CTAGTAATCGCGGGTCAGCATACCGCGGTGAATACGTTCCC GGGCCTTGTACACACCGCCCGTCACACTATGAGAGTCAGA AACACCCGAAGCCGGTGAGGTAACCGCAAGGAGCCAGCCG TCGAAGGCGAG |

Table 2 shows the plurality of predetermined microbial marker sequences associated with the saliva sample. As shown in table 2, the plurality of predetermined microbial marker sequences associated with the saliva sample are listed from Sal_seq1 to Sal_seq9 in the form of nucleic acid sequences.

Table 2

| Sequence ID | Microbial marker sequences for saliva sample |
|---|---|
| Sal_seq1 | GAGTTTGATCCTGGCTCAGGGTGAACGCTAGCGGTGCGCC TAACACATGCAAGTCGAGCGGAATTGATATGTTGAAAGCT TCGGTGGGAAATATATTGAGAGAGCGGCGAACGGGTGAG GAACACGTTGGAATCTGCCCCCAAGTCAGGGATAGCCCAG GGAAACCTGGATTAATACCGGATAGTCTCTTTGGAGTAAA GATTTATTGCTTGGGGAGGAGCCTGCGTACTATCAGCTAG TTGGTAGGGTAAAAGCCTACCAAGGCAATGACGGATAAC TGGTCTGAGAGGATGATCAGTCACAATGGAACTGAGACA CGGTCCATACTCCTACGGGAGGCAGCAGTGAGGAATCTTC CACAATGGGCGAAAGCCTGATGGAGCGACACCGCGTGAA GGAAGAAGGCCTAACGGTTGTAAACTTCTTTTCTGAAGGA GCATAATGAGAGTACTTTAGGAATAAGGGACGGCTAAAT |

(continued)

| Sequence ID | Microbial marker sequences for saliva sample |
|---|---|
| | ACGTGCCAGCAGCCGCGGTAATACGTATGTCTCGAGCGTT ACCCGGAATTACTGGGTGTAAAGGGTTTGTAGGTTGGTGA ATAAGTCAGGTATGAAAGACCGGAGCTTAACTCCGAGTTT GTGCTTGAAACTGTTGACCTAGAATCAGGGAGAGGTAAGC GGAATTCTAAGTGTAGGGGTGCAATCCGTAGATACTTAGA GGAACACCAAAAGCGAAGGCAGCTTACTGGAACTGCATT GACACTGAAAAACGAAAGCGTGGGTAGCGAAAAGGATTA GATACCCTTGTAGTCCACGCCCTAAACGATGATAACTAAG TGTTGCGACGAGCTCGCAGTGCTGTAGCAAACGCGTTAAG TTATCCACCTGAGGAGTACGGTCGCAAGATTAAAACTCAA AGGAATAGACGGGGACCCACACAAGCAGTGGATCACGTG GTTTAATTCGACAATAAACGGGGAACCTTACCCAGACTTG ACATCCTAGGAATACTTTAGAGATAGAGTAGTGCTCGCAA GAGAACCTAGAGACAGGTGCTGCATGGTTGTCGTCAGCTC GTGCCTTGAGGTGTTCGGTTAAGTCCGTTAACGAGCGCAA CCCATGTCCTTAGTTACAATGTCTAAGGAGACTGCCTTGG TTAACAAGGAGGAAGGTGTGGATGACGTCAAATCAGCAT GGCTTTTACGTCTGGGGCTACACACATGATACAATGGCCT GTACAAGAGTAGTGAAACCGCGAGGTAGAACCAATCTT GAAAGCAGGTCTCAGTCCGGATTGGAGTCTGCAACTCGAC TCCATGAAGTTGGAATTGCTAGTAATCGTAAATCAGATAT GTTACGGTGAATGTGTTCCTGGGTCTTGTACTCACCGCCCG TCAAGGCATGGGAGGTAGTAATATCGGAAGTCCCCCTAGA TATAAGGGGGCCCATGGTAGGACTACTGACTGGGCTTAAG TCGTAACAAGG |
| Sal_seq2 | ACGCTAGCGGTGCGCCTAACACATGCAAGTCGAGCGAGA GTTTTTGAGAAGCTTGCTTCTTGAAAACAAAAGCGGCGAA CGGGTGAGTAACACGTTGGAATCTGCCCCTAAGTCAGGGA |

(continued)

| Sequence ID | Microbial marker sequences for saliva sample |
|---|---|
| | TAGCCTAGTGAAAACTAGATTAATACCGGATAGTCTCTTT GGAGTAAAAATTTATTGCTTGGGGAGGAGCCTGCGTTCTA TCAGCTAGTTGGTGAGGTAAGAGCTTACCAAGGCTATGAC GGATAACTGGTCTGAGAGGATGATCAGTCACAATGGAACT GAGACACGGTCCATACTCCTACGGGAGGCAGCAGTGAGG AATCTTCCACAATGGACGAAAGTCTGATGGAGCGACACCG CGTGAAGGATGAAGGCCTAACGGTTGTAAACTTCTTTTCT GAAGGAGCATAATGAGAGTACTTTAGGAATAAGGGACGG CTAAATATGTGCCAGCAGCCGCGGTAATACATATGTCTCA AGCGTTACCCGGAATCACTGGGTGTAAAGGGTTTGTAGGT GGTTTTTTAAGTCAGGTATGAAAGACCGGAGCTCAACTCC GAGTTTGTATTTGAAACTGGAGAACTAGAATCAGGGAGA GGTAAGCGGGATTCTAAGTGTAGGGGTGCAATCCGTAGAT ACTTAGAGGAACACCAAAAGCGAAGGCAGCTTACTGGAA CTGTATTGACGCTGAGAAACGAAAGCGTGGGGAGCGAAA AGGATTAGATACCCTTGTAGTCCACGCCCTAAACGATGAC AACTAAGTGTTGCGACTAGCTCGCAGTGCTGTAGCTAACG CGTTAAGTTGTCCACCTGAGGAGTACGGTCGCAAGATTAA AACTCAAAGGAATAGACGGGGACCCACACAAGCAGTGGA TCACGTGGTTTAATTCGATAATAAACGGGGAACCTTACCC AGACTTGACATCCTAAGAATGTTCTAGAGATAGAATAGTG CTCGCAAGAGAGCTTAGAGACAGGTGCTGCATGGTTGTCG TCAGCTCGTGCCTTGAGGTGTTCGGTTAAGTCCGTTAACG AGCGCAACCCATGTCCTTAGTTACAATGTCTAAGGAGACT GCCTTGGTTAACAAGGAGGAAGGTGTGGATGACGTCAAA TCAGCATGGCTTTTACGTCTGGGGCTACACACATGATACA ATGGTATGTACAAAGAGTAGTGAAATCGCGAGATAGAAC CAATCTTAAAAACATATCTCAGTCCGGATTGGAGTCTGCA |

(continued)

| Sequence ID | Microbial marker sequences for saliva sample |
|---|---|
| | ACTCGACTCCATGAAGTTGGAATTGCTAGTAATCGTAAAT CAGATATGTTACGGTGAATATGTTCCTGGGTCTTGTACTCA CCGCCCGTCAAGGCATGGGAGGTAGTAATATCGGAAGTCC CCCTGGTAATATGGGGGCCCATGGTAGGACTACTGACTGG GCTTAAGTCGTAACAAGGTAGCCGTA |
| Sal_seq3 | AGAGTTTGATAATGGCTCAAGATTAACGCTGGCGGCGTGC CTAACACATGCAAGTCGAGCGGCAGCGAGTTTTTACACTG AATTCTGGAAGCTTCTAGTTGAAAGAGAAGATTTATTCAA GAATTTTGTGTAAAATGTCGGCGAGCGGCGGACGGCTGAG TAACGCGTGGGAACGTACCCCAAAGTGAGGGATAACGCA TCGAAAGGTGTGCTAATACCGCATATGGTCTTCGGATTAA AGCCTTCGGGCGCTTTGGGAACGGCCTGCGTAAGATTAGA TAGTTGGTGGGGTAATGGCCTACCAAGTCGACGATCTTTA ACTGGTTTGAGAGGATGATTCAGTCAGACTGGAACTGAGA CACGGTCCAGACTCCTACGGGAGGCAGCAGTGAGGAATC TTCCACAATGGGCGAAAGCCTGATGGAGCAACGCCGCGT GCAGGATGAAGGCCTTCGGGTTGTAAACTGCTTTTATAAG TGATGATTATGACAGTAACTTATGAATAAGGATTCGGCTA ACTACGTGCCAGCAGCCGCGGTCATACGTAGGATCCGAGC GTTATCCGGAGTGACTGGGCGTAAAGAGTTGCGTAGGTGG TTTAATAAGCGAATAGTGAAATCTGGTGGCTCAACCATTC AGACTATTATTCGAACTGTTGAACTCGAGAGCAGAAGAGG TAGCTGGAATTTCTAGTGTAGGAGTGAAATCCGTAGATAT TAGAAGGAACACCGATGGCGTAGGCAGGCTACTGGGCTG TTTCTGACACTGAGGCACGAAAGCGTGGGGAGCGAGCCG GATTAGATACCCGGGTAGTCCACGCCGTAAACGATGGATA CTAGCTGTTTGGGGTATCGACCCCCTGAGTAGCGAAGCTA |

(continued)

| Sequence ID | Microbial marker sequences for saliva sample |
|---|---|
| | ACGCGTTAAGTATCCCGCCTGTGGAGTACGATCGCAAGAT TAAAACATAAAGGAATTGACGGGGACCCGCACAAGCGGT GGATCATGTTCTTTAATTCGATGATAACCGATAAACCTTA CCAGGTCTTGACATCCTTGGAATCTTTCAGAAATGAGAGA GTGCTTTTTAAGAGCCAAGTGACAGGTGATGCATGGCCGT CGTCAGCTCGTGTCGTGAGATGTTTGGTTAAGTCCATCAA CGAGCGCAACCCTTGTCAGTAGTTGTATTTTTCTACTGAGA CTGCCCCGGTAACGGGGAGGAAGGAGGGGATGACGTCAG GTCAGTATTTCCCTTACGTCCTGGGCTAGAAACGTGATAC AATGGCTAGTACAATGCGCAGCGAAGCCGCGAGGTGAAG CAAATCGCATCAAAGCTAGTCCCAGTTCGGATTGTAGGCT GAAAACTCGCCTGCATGAAGTCGGAATCGCTAGTAATCGC GGTTCAGCTTGCTGCGGTGTATACGTTCCCGGG |
| Sal_seq4 | TTTGATCCTGGCTCAGGATGAACGCTAGCGAAATGCCTAA CACATGCAAGTCGAACGGATCTTGCGTCGTAGCAATACGT AGTAAGATTAGTGGCAAACGAGCGAGTAACGCGTGCTTA ACTTGCCCCGAAGATGGGTATAACTACAGAGATGTAGCTA ATTCCCAATATGCTCACGATGGTGAAATTCCAATGTGAGG AAAGATTTATCGCTTCGGGAGAGTGGTGCGTCCTATCAGG TAGTTGGTAAGGTAATGGCTTACCAAGCCTATGACGGGTA ACCGGTCTGAGATGATGTCCGGTCGCGATGGGACTGAGAT ACGGCCCATACTCCTACGGGAGGCAGCAGTGGGGAATCTT GCACAATGGACGAAAGTCTGATGCAGCAATTTCGCGTGAA GGATGAAGCATTACGGTGTGTAAACTTCTTTTTTGGCAGA AGACGAATGACGGTATGTCAAGAATAAGAGACGGCTAAC TACGTGCCAGCAGCCGCGGTAATACGTAGGTCTCAAGCGT TGTCCGGATTTACTGGGCGTAAAGTGTCCGTAGTCTGAAT TGTAAGTCTGTTTTCAAATCCTACGACTCAATCGTAGAAA |

(continued)

| Sequence ID | Microbial marker sequences for saliva sample |
|---|---|
| | GGGAGTGGATACTGCAATTCTGGAAGTATCTGGGGGTTAG TGGAATTTCCGGTGGAGCGGTGAAATGCGTTGATATCGGA AGGAACGCCGAAAGCGAAAGCAGCTAACTACAGAATACT TGACGATGAGGGACGACAGTTCGGGTAGCAAACAGGATT AGATACCCTGGTAGTCCGAACCGTAAATTATGCTTGCTAG ATGTTTTTGTCAATTTATTGGCAGGAGTGTCGTAATCTAAC GAGTTAAGCAAGTCGCCTGGGTAGTATATTCGCAAGAATG AAACTCAAAGGGATAGGCGGGGGAACACACAAGCAGTGG ATTATCTAGATTAATTGGATAATAAGCCAAGAATCTTACC TAGGATTGACATGTATTGTGTCTGCGGTGAAAGTCGCATA TCCGTAGCAATACGGAGCTTTACACAGATGGTGCATGGTC GTCGTCAGCTCGTGCCGCAAGGTGTCTAGTTAAGTCTGGA AACGAGCGCAACCCTCATGCTTAGTTAGTATGTCTAAGCA GACTGCTCGGGTAACCGAGAGGAAGGAGAGGATGACGTC AGATCCTCATGCCTCTTACACCTAGGGCCTCATAGATAAT ACAATGGGTAGGTACAGCGAGAAGCAAGACCGCGAGGTG GAGCAAATCTTTAAAACTACCCCTAGTTCGGATTGTAGTC TGGAACTCGACTACATGAAGTTGGAATTGCTAGTAATGGC AGATCAGCCATGCTGCCGTGAATATGTCCCTGTTCCTTGC ACACACCGCCCGTC |
| Sal_seq5 | GAGTTTGATCCTGGCTCAGGATGAACGCTGGCGGCGTGCC TAACACATGCAAGTCGAGCGGCAGCGAGTTTTACACTAAG TTCTGGAAGTGATTCGAGAATTTTGTGTAAAATGTCGGCG AGCGGCGGACGGCTGAGTAGCGCGTGGGAACATACCCCA AAGTGAGGGATAACTGCCCGAAAGGGTGGCTAATACCGC ATATGATCTTCGGATTAAAGGATTTATCCGCTTTGGGAAT GGCCTGCGTCGGATTAGGTAGTTGGTGAGGTAAAGGCTCA CCAAGCCGACGATCCGTAACTGGTTTGAGAGGATGACCAG |

(continued)

| Sequence ID | Microbial marker sequences for saliva sample |
|---|---|
| | TCAGATTGGAACTGAGACACGGTCCAGACTCCTACGGGAG GCAGCAGTGAGGAATCTTCCACAATGGGCGAAAGCCTGA TGGAGCAACGCCGCGTGCAGGATGAAGGCCTTCGGGTTGT AAACTGCTTTTATGATTGAGGAATTTGACGGTAGATCATG AATAAGGATCGGCTAACTACGTGCCAGCAGCCGCGGTCAT ACGTAGGATCCGAGCGTTATCCGGAGTGACTGGGCGTAAA GAGTTGCGTAGGTGGTTTATTAAGTAGGTGATGAAAGCTG GTGGCTCAACCATTCAGATTGTTATCTAAACTGGTAAACT TGAGAGTAGTAGAGGTAACTGGAATTTCTAGTGTAGGGGT AAAATCCGTAGATATTAGAAGGAACACCAATGGCGTAGG CAGGTTACTGGACTATTTCTGACACTGAGGCACGAAAGCG TGGGGAGCGAACGGGATTAGATACCCCGGTAGTCCACGC CCTAAACGATGGATACTAGCTGTTTGAGGAATCGACCCCT TAAGTAGCGAAGCTAACGCGTTAAGTATCCCGCCTGTGGA GTACGATCGCAAGATTAAAACATAAAGGAATTGACGGGG ACCCGCACAAGCGGTGGAGCATGTTCTTTAATTCGATGCT AATCGATATACCTTACCAAGGCTTGACATCTCGGGAAGGC CTCCGAAAGGAGACTGTGCCTTTTAGGAACCCGATGACAG GTGATGCATGGCCGTCGTCAGCTCGTGTCGTGAGATGTTT GGTTAAGTCCATTAACGAGCGCAACCCTTGCAACTAGTTG GATTTTTCTAGTTGGACTGCCCCGGTAACGGGGAGGAAGG AGGGGATGATGTCAGGTCATTATTTCCCTTACGCCTTGGG CTAGAAACGTGCTACAATGGCTGGTACAAAGTGCATAAGC GAACTCGCGAGAGCAAGCAAATCACATCAAAACCAGTCC CAGTTCGGATTGGAGGCTGAAACTCGCCTCCATGAAGTCG GAATCGCTAGTAATCGTAAATCAGCAAGTTACGGTGAATA CGTTCCCGGGTCTTGTACACACCGCCCGTCAAACCATGAG AGTGACCAACACCCGAAGTCCGATTCGTCGGCCTAAGGCG GGGGGCATGATTGGGGTTAAGTCGTAACAAGGTA |

(continued)

| Sequence ID | Microbial marker sequences for saliva sample |
|---|---|
| Sal_seq6 | AGAGTTTGATCATGGCTCAGGACGAACGCTGGCGGCGTGC<br>TTCATACATGCAAGTCGAACGAGAATCTGGTGCTTGCACC<br>AGAGGAAAGTGGCGGACGGGTGAGTAATATGTAGGAAAT<br>CTGCCCTAGAGAGGGGGACAACAGAGGGAAACTTCTGCT<br>AATACCCCATATGAGCGTACCTGAAATGGTATTCTTGAAA<br>ACTCCGGTGCTCTAGGATGAGCCTGCATCTGATTAGTTTGT<br>TGGCGGTGTAATGGACCACCAAGACTACGATCAGTAGCTG<br>GTTTGAGAGGATGATCAGCCACAATGGGACTGAGACACG<br>GCCCATACTCCTACGGGAGGCAGCAGTAGGGAATTTTGCG<br>CAATGGGCGAAAGCCTGACGCAGCAACGCCGCGTGTGTG<br>ATGACGCCCTTCGGGGTGTAAAACACTGTCAGTAGGGACG<br>AAACTTGACGGTACCTACAGAGGAAGCACCGGCTAACTCC<br>GTGCCAGCAGCCGCGGTAATACGGAGGGTGCAAGCGTTG<br>TCCGGAATCATTGGGCGTAAAGAGTTCGTAGGTGGTTTGT<br>TAAGTCTGGTGTTAAAGCCCGAAGCTCAACTTCGGTTCGG<br>CATCGGATACTGGCAGACTAGAATGCGGTAGAGGTAAAG<br>GGAATTCCTGGTGTAGCGGTGAAATGCGTAGATATCAGGA<br>GGAACATCGGTGGCGTAAGCGCTTTACTGGGCCGTAATTG<br>ACACTGAGGAACGAAAGCCGGGGTAGCAAATGGGATTAG<br>ATACCCCAGTAGTCCCGGCCGTAAACGATGGATACTAGGT<br>GTTGCGGGTATCGACCCCTGCAGTGCCGTAGTTAACGCGA<br>TAAGTATCCCGCCTGGGGAGTACGCACGCAAGTGTGAAAC<br>TCAAAGGAATTGACGGGGACCCGCACAAGCGGTGGAACA<br>TGTGGTTTAATTCGAAGCAACGCGAAGAACCTTACCAGGG<br>CTTGACATCTGAGGAACCTTTGTGAAAGCAGAGGGTGCTC<br>TTCGGAGAACCTCAAGACAGGTGGTGCACGGTTGTCGTCA |

(continued)

| Sequence ID | Microbial marker sequences for saliva sample |
|---|---|
|  | GCTCGTGTCGTGAGATGTTGGGTTAAGTCCCGCAACGAGC GCAACCCTCGTCGTTAGTTGCATATATTGGTATACTGATAT ATTGCTCTCTAGCGAGACTGCCGGTGATAAACCGGAGGAA GGTGGGGACGACGTCAAATCATCATGCCCCTTATGTCCTG GGCTACACACGTGTTACAATGGCTAAGACAACGAGCCGCC AACTCGCGAGAGTGAGCAAATCTCTTAAACTTAGTCTCAG TTCGGATTGCACTCTGCAACTCGAGTGCATGAAGTCGGAA TCGCTAGTAACCGTAGATCAGCACGCTGCGGTGAATACGT TCCCGGGTCTTGTACACACCGCCCGTCACACCATGGAAGT CGACCACGCCCGAAGCACGTGGGCTAACCTTTTGGAGGCA GCGTTCTAAGGCAGGGTTGGTGACTGGGGTGAAGTCGTAA CAAGGTAGCCGTACCGGAGGGTGTGGCTGGATCACCTCCT T |
| Sal_seq7 | AGAGTTTGATCCATGGCTCAGGATGAACGCTGGCGGCGTG CCTAACACATGCAAGTCGAGCGGCAGCGCGAGTAGTTTAC TACTTGGCGGCGAGCGGCGGACGGCTGAGTAACGCGTGG GAATATACCCCAAAGTGAGGGATAACTGCCCGAAAGGGT AGCTAATACCGCATATGATCTTCGGATTAAAGGATTTATC CGCTTTGGGAGTGGCCCGCGTCGGATTAGGTAGCTGGTGA GGTAATGGCTCACCAAGCCGACGATCCGTAGCTGGTCTGA GAGGATGATCAGCCAGACTGGGACTGAGACACGGCCCAG ACTCCTACGGGAGGCAGCAGTGAGGAATCTTCCACAATGG GCGAAAGCCTGATGGAGCAACGCCGCGTGCAGGATGAAG GCCTTCGGGTTGTAAACTGCTTTTATAAGTGAGGAATATG ACGGTAACTTATGAATAAGGATCGGCTAACTACGTGCCAG CAGCCGCGGTCATACGTAGGATCCGAGCGTTATCCGGAGT GACTGGGCGTAAAGAGTTGCGTAGGCGGTTTGTTAAGCGA ATAGTGAAACCTGGTGGCTCAACCATACAGACTATTATTC |

(continued)

| Sequence ID | Microbial marker sequences for saliva sample |
|---|---|
|  | GAACTGGCAAACTCGAGAGTGGTAGAGGTCACTGGAATTT CTTGTGTAGGAGTGAAATCCGTAGATATAAGAAGGAACA CCGATGGCGTAGGCAGGTGACTAGACCATTTCTGACGCTA AGGCACGAAAGCGTGGGGAGCGAACCGGATTAGATACCC GGGTAGTCCACGCCGTAAACGATGGATACTAGCTGTTGGA GGTATCGACCCCTTCAGTAGCGAAGCTAACGCGTTAAGTA TCCCGCCTGTGGAGTACGGCCGCAAGGCTAAAACATAAA GGAATTGACGGGGACCCGCACAAGCGGTGGATCGTGTTCT TTAATTCGATGATAAACGAAGAACCCTACCAGGGCTTGAC ATCCAGGGAAGGTCTGCGAAAGCGGACTGTGCCTTTTGGA ACCCTGTGACAGGTGCTGCATGGCCGTCGTCAGCTCGTGT CGTGAGATGTTTGGTTAAGTCCATCAACGAGCGCAACCCT TGCAACTAGTTGGATTTTTCTAGTTGGACTGCCCCGGTAAC GGGGAGGAAGGAGGGGATGATGTCAGGTCAGTATTGCCC TTACGTCCTGGGCTAGAAACACGATACAATGGCTAGTACA ATGCGCAGCGAAGCCGCGAGGTGGAGCAAATCGCATCAA AGCTAGTCCCAGTTCGGATTGGAGGCTGAAACTCGCCTCC ATGAAGTCGGAATCGCTAGTAATCGCAGATCAGCAAGCTG CGGTGAATACGTTCCCGGGTTCTTGCACACACCGCCCGTC AAA |
| Sal_seq8 | TTTGATCATGGCTCAGGATGAACGCTGGCGGCGTGCCTAA CACATGCAAGTCGAGCGGCAGCGCGAGTAGTTTACTACTT TGGCGGCGAGCGGCGAACGGCTGAGTAACGCGTCGGGAA TTTGCCCCAAAGTGAGGAAATAACTGCCCGAAAGGGTCG GCTAATGCCGCATATGATTCTTCGGAATAAAGGAATTATC CGCTTTTGGGAGAAACCCGCGTCGGATTAGGTAGTTTGGT GAGGTAATGGCTCACCAAGCCGACGAACCGTAGCTGGTCT GAGAGGATGACCAGCCAGACTGGAACTGAGACACGGTCC |

(continued)

| Sequence ID | Microbial marker sequences for saliva sample |
|---|---|
|  | AGACTCCTACGGGAGGCAGCAGTGAGGAATCTTCCACAAT GGGCGAAAGCCTGATGGAGCAACGCCGCGTGCAGGATGA AGGCCTTCGGGTTTGTAAACTGCTTTTATGATTGAGGAATT TTGACAGTAGATTCATGAATAAGGATCGGCTAACTACGTG CCAGCAGCCGCGGTCATACGTAGGATTCCGAGCGTTATCC GGAGTGACTGGGCGTAAAGAGTTGCGTAGGCGGTTTTGTT AAGTGAATAGTGAAATCTGGTGGCTCAACCATACAGGCTA TTATTCAAACTGGCAAACTCGAGAGTGGTAGAGGTCACTG GAATTTCTTGTGTAGGAGTGAAATCCGTAGATATAAGAAG GAACACCGATGGCGTAGGCAGGTGACTGGACCATTTCTGA CGCTAAGGCACGAAAGCGTGGGGAGCGAACCGGATTAGA TACCCGGGTAGTCCACGCCGTAAACGATGGATACTGGCTG TTGGAGGTATCGACCCCTTCAGTAGCGAAGCTAACGCGTT AAGTATCCCGCCTGTGGAGTACGGTCGCAAGACTAAAACA TAAAGGAATTGACGGGGACCCGCACAAGCGGTGGATCGT GTTCTTTAATTCGATGCTGAACGAAGAACCTTACCAGGGC TTGACATCCAGGGAATTTTTGGGAAACCAATTAGTGCCTT TTGGAACCCTGTGACAGGTGATGCATGGCCGTCGTCAGCT CGTGTCGTGAGATGTTTGGTTAAGTCCATCAACGAGCGCA ACCCTTGCAACTAGTTGGATTTTTCTAGTTGGACTGCTTCG GTAACGGAGAGGAAGGAGGGGATGATGTCAGGTCAGTAT TTCCCTTACGTCCTGGGCTAGAAACACGATACAATGGCTA GTACAATGCGCAGCAAAGCCGCGAGGTGGAGCAAACCGC ATCAAAGCTAGTCCCAGTTCGGATTGGAGGCTGAAACTCG CCTCCATGAAGTCGGAATCGCTAGTAATCGCAGATCAGCA AGCTGCGGTGAATACGTTCCCGGGTCTTGTACACACCGCC CGTC |

(continued)

| Sequence ID | Microbial marker sequences for saliva sample |
|---|---|
| Sal_seq9 | AGAGTTTGATCCTGGTTCAGGATGAACGCTGGCGGCATGC CTAACACATGCAAGTCGATCGGTAAGGCCCTTCGGGGTAC ACGAGAGGCGGACGGCTGAGTAACGCGTGGGAACGCACC CTACACTGAGGGATAAGATACCGAAAGGTGTTCTAATACC GCATACGGTCTTCGGATTAAAGTCTTCGGACGGTGAAGGA GCGGCCCGCGTCATATTAGGTAGTTGGTGGGGTAATGGCC TACCAAGCCGATGATGTGTAGCTGGTCTGAGAGGATGATC AGCCAGACTGGAACTGAGAACGGTCCAGACTCCTACGGG AGGCAGCAGTGAGGAATATTCCACAATGGGCGAAAGCCT GATGGAGCAATGCCGCGTGCAGGATGAAGGCCCTCGGGT CGTAAACTGCTTTTATTAGAGAAGAATATGACGGTAACTA ATGAATAAGGGACGGCTAACTACGTGCCAGCAGCCGCGG TCATACGTAGGTCCCAAGCGTTATCCGGAGTGACTGGGCG TAAAGAGTTGCGTAGGCGGCTAAGTAAGCGAGTAATGAA AACTATCGGCTCAACCGGTAGCCTGTTATTCGAACTGCTT GGCTCGAGATTATCAGAGGTCGCTGGAATTCCTAGTGTAG CAGTGAAATGCGTAGATATTAGGAAGAACACCAATGGCG TAGGCAGGCGACTGGGGTATTTCTGACGCTAAGGCACGAA AGCGTGGGGAGCGAACCGGATTAGATACCCGGGTAGTCC ACGCCGTAAACGATGGATGCTAATTGTTCGGGGTATCGAC CCCTTGAGTAATAAAGCTAACGCGTTAAGCATCCCGCCTG TGGAGTACGGCCGCAAGGCTAAAACATAAAGGAATTGAC GGGGACCCGCACAAGCGGTGGAGGATGTTCTTTAATTCGA TGATAAGCGAAGAACCTTACCAGGGCTTGACATCCCTAGA ATTTCTCCGAAAGGAGAGAGTGCTTTTTAAGAACTAGGTG ACAGATCCTGCATGGCCGTCGTCAGCTCGTGTCGTGAGAT GTTTGGTTAAGTCCATCAACGAGCGCAACCCTTATCGTTA GTTGTATTTTTCTAACGAGACTGCCCCGGTAACGGGGAGG |

(continued)

| Sequence ID | Microbial marker sequences for saliva sample |
|---|---|
| | AAGGAGGGGATGATGTCAGGTCAGTATTGGTCTTACGTCC |
| | TGGGCTAGAAACGTCCTACAATGGCTAGTACAATGGGCAG |
| | CGAATCCGCGAGGTGAAGCAAATCCCATCAAAGCTAGTCC |
| | CAGTTCGGATTGCAGGCTGAAACTCGCCTGCATGAAGTCG |
| | GAATCGCTAGTAATCGCAGATCAGCACGCTGCGGTGAATA |
| | CGTTCCCGGGTCTTGTACACACCGCCCGTCAAACCATGAA |
| | AGTCAGGAGCACCCGACGTCCGAATTTATTTCGGCCTAAG |
| | GTGAACTTGGTGATTGGGGTTCAGTCGTAACAAGGTAACC |

The quantitative abundance of each of the plurality of predetermined microbial marker sequences associated with the biological sample, are determined using a set of probes specific to each of the plurality of predetermined microbial marker sequences associated with the corresponding biological sample. The set of probes includes a plurality of probes where each probe is utilized for each of the plurality of predetermined microbial marker sequences (one probe for one predetermined microbial marker sequence) associated with the corresponding biological sample.

In an embodiment, a multiplexed quantitative Polymerase Chain Reaction (qPCR) technique is employed for determining the quantitative abundance. More specifically, the multiplexed quantitative Polymerase Chain Reaction (qPCR) technique define a design/layout and an arrangement of the plurality of probes that are used for determining the quantitative abundance associated with the corresponding biological sample.

More specifically, the set of probes specific to each of the plurality of predetermined microbial marker sequences associated with the stool sample are utilized in five sequential multiplexed qPCR runs (defined by the multiplexed quantitative Polymerase Chain Reaction (qPCR) technique), to determine the quantitative abundance of each of the plurality of predetermined microbial marker sequences associated with the stool sample.

FIG. 3A illustrates an exemplary probe and multiplexed qPCR design for detecting and determining the quantitative abundance of each of the plurality of predetermined microbial marker sequences associated with the stool sample, according to some embodiments of the present disclosure. As shown in FIG. 3A, the five sequential multiplexed qPCR runs namely a first multiplexed qPCR run (Run 1), a second multiplexed qPCR run (Run 2), a third multiplexed qPCR run (Run 3), a fourth multiplexed qPCR run (Run 4), and a fifth multiplexed qPCR run (Run 5) are defined for determining quantitative abundance of each of the plurality of predetermined microbial marker sequences associated with the biological sample being the stool sample.

Each run of the first multiplexed qPCR run (Run 1), the second multiplexed qPCR run (Run 2), the third multiplexed qPCR run (Run 3), the fourth multiplexed qPCR run (Run 4), and the fifth multiplexed qPCR run (Run 5) includes five probes (hence it is also called as five-plex qPCR run) where each probe is utilized for one predetermined microbial marker sequence associated with the stool sample, from the list of Gut_seq1 to Gut_seq15 listed in table 1. Further, each run of the five multiplexed qPCR runs contains a non-specific probe (denoted as 'Z' in FIG. 3A) at the start.

Further, as shown in FIG. 3A, the plurality of predetermined microbial marker sequences, the quantitative abundance of which are being determined through the first multiplexed qPCR run (Run 1) are: Gut seq1, Gut_seq2, Gut_seq3, and Gut_seq4 listed in table 1. The plurality of predetermined microbial marker sequences, the quantitative abundance of which are being determined through the second multiplexed qPCR run (Run 2) are: Gut seq1, Gut_seq5, Gut_seq6, and Gut_seq7 listed in table 1. The plurality of predetermined microbial marker sequences, the quantitative abundance of which are being determined through the third multiplexed qPCR run (Run 3) are: Gut_seq8, Gut_seq5, Gut_seq9, and Gut seq10 listed in table 1. The plurality of predetermined microbial marker sequences, the quantitative abundance of which are being determined through the fourth multiplexed qPCR run (Run 4) are: Gut_seq8, Gut_seq11, Gut_seq12, and Gut_seq13 listed in Table 1. Lastly, the plurality of predetermined microbial marker sequences, the quantitative abundance of which are being determined through the fifth multiplexed qPCR run (Run 5) are: Gut_seq6, Gut_seq11, Gut_seq14, and Gut_seq15 listed in table 1.

Similarly, the set of probes specific to each of the plurality of predetermined microbial marker sequences associated with the saliva sample are utilized in three sequential multiplexed qPCR runs (defined by the multiplexed quantitative Polymerase Chain Reaction (qPCR) technique), to determine the quantitative abundance of each of the plurality of predetermined microbial marker sequences associated with the saliva sample.

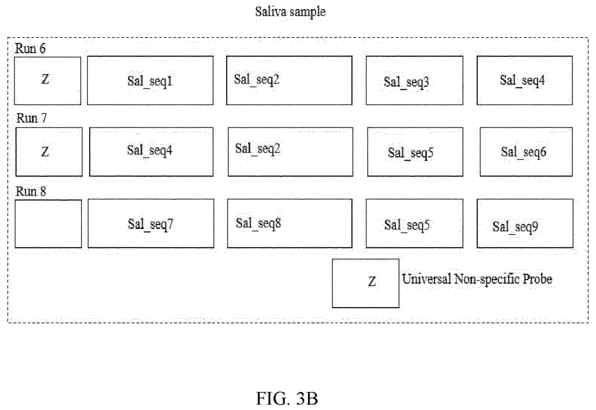

FIG. 3B illustrates an exemplary probe and multiplexed qPCR design for detecting and determining the quantitative abundance of each of the plurality of predetermined microbial marker sequences associated with the saliva sample, according to some embodiments of the present disclosure. As shown in FIG. 3B, the three sequential multiplexed qPCR runs namely a sixth multiplexed qPCR run (Run 6), a seventh multiplexed qPCR run (Run 7), and an eighth multiplexed qPCR run (Run 8) are defined for determining the quantitative abundance of each of the plurality of predetermined microbial marker sequences associated with the biological sample being the saliva sample.

Each run of the sixth multiplexed qPCR run (Run 6), the seventh multiplexed qPCR run (Run 7), and the eighth multiplexed qPCR run (Run 8) includes five probes (hence it is also called as five-plex qPCR run) where each probe is utilized for one predetermined microbial marker sequence associated with the saliva sample, from the list of Sal_seq1 to Sal_seq9 listed in table 2. Further, each run of the three multiplexed qPCR runs (Run 6, Run 7, and Run 8) contains a non-specific probe (denoted as 'Z' in FIG. 3B) at the start.

Further, as shown in FIG. 3B, the plurality of predetermined microbial marker sequences, the quantitative abundance of which are being determined through the sixth multiplexed qPCR run (Run 6) are: Sal_seq1, Sal_seq2, Sal_seq3, and Sal_seq4 listed in table 2. The plurality of predetermined microbial marker sequences, the quantitative abundance of which are being determined through the seventh multiplexed qPCR run (Run 7) are: Sal_seq4, Sal_seq2, Sal_seq5, and Sal_seq6 listed in table 2. Lastly, the plurality of predetermined microbial marker sequences, the quantitative abundance of which are being determined through the eighth multiplexed qPCR run (Run 8) are: Sal_seq7, Sal_seq8, Sal_seq5, and Sal_seq9 listed in table 2.

In an embodiment, the plurality of predetermined microbial marker sequences associated with the stool sample and the plurality of predetermined microbial marker sequences associated with the saliva sample are identified from respective microbes, where the respective microbes are captured from features of the respective pre-determined machine learning (ML) model. In an embodiment, the pre-determined machine learning (ML) model associated to the saliva sample is an ensemble machine learning (ML) model that is built using a microbial abundance data corresponding to a plurality of training saliva samples. The plurality of training saliva samples are the saliva samples used for training a machine learning model to obtain the corresponding pre-determined machine learning (ML) model. In an embodiment, the microbial abundance data corresponding to the plurality of training saliva samples is the quantitative abundance of all the microbial marker sequences (associated with the microbes) present in each of the plurality of training saliva samples.

Similarly, the pre-determined machine learning (ML) model associated to the stool sample is an ensemble machine learning (ML) model that is built using the microbial abundance data corresponding to a plurality of training stool samples. The plurality of training stool samples are the stool samples used for training the machine learning model to obtain the corresponding pre-determined machine learning (ML) model. In an embodiment, the microbial abundance data corresponding to the plurality of training stool samples is the quantitative abundance of all the microbial marker sequences (associated with the microbes) present in each of the plurality of training stool samples.

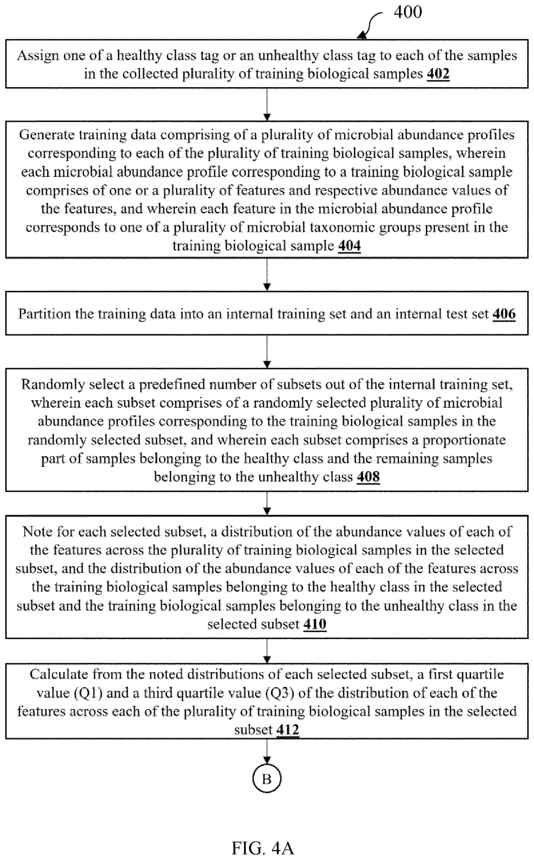

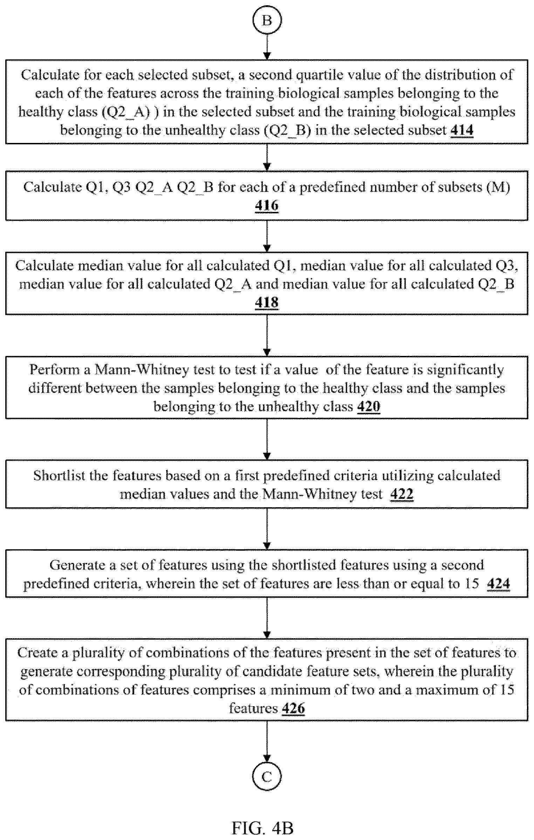

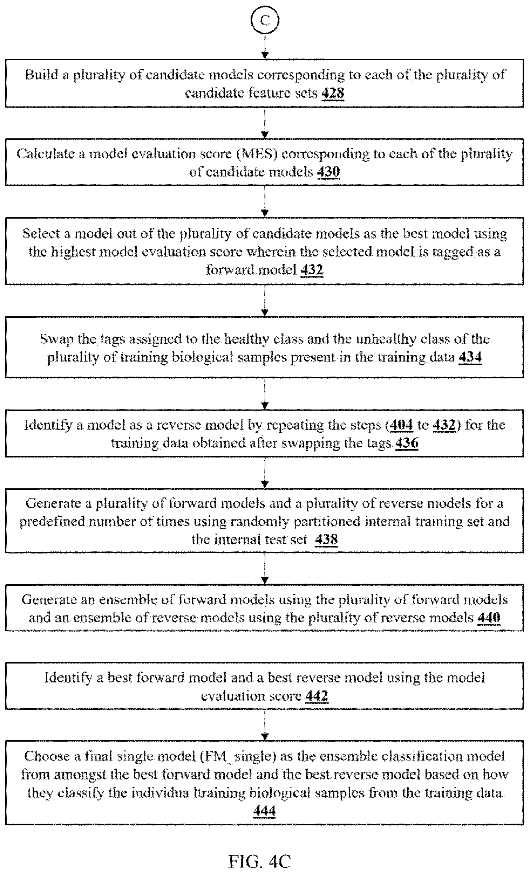

The ensemble ML model is built using the plurality of training biological samples consisting of either the saliva samples or the stool samples, individually, to obtain the corresponding pre-determined machine learning model. FIG. 4A, 4B and 4C are flowcharts illustrating steps 400 involved in building a pre-determined machine learning model according to some embodiments of the present disclosure. The technique (400) for building the ensemble ML model accepts data in form of a feature table for multiple observations (the plurality of training biological samples) wherein each observation/training biological sample is defined by 'N' features (F) which are either or both of continuous and counted variables and ($N \geq 1$). In case of training data (TR), each of the training biological samples/observations further have a preassigned class/category which is binary in nature, i.e., a healthy class (A) (e.g., affiliating to samples sourced from pregnant women who delivered a baby/ babies after at least 37 weeks of gestation (i.e., a "term" delivery)) and an unhealthy class or diseased class (B) (e.g., affiliating to samples sourced from pregnant women who delivered (PTD) a "preterm" baby/ babies prior to completing 37 weeks of gestation). In case of test data (TS) or data received during actual deployment of the method, the model(s) built based on training data predicts the class/category of the training biological samples/observations. During training process, the following steps are followed:

Initially at step 402, a healthy class tag or an unhealthy class tag is assigned to each of the training biological samples in the collected plurality of training biological samples (either saliva samples or stool samples). The healthy class tag indicates samples sourced from pregnant women who delivered a baby (or babies) after at least 37 weeks of gestation (i.e., a "term" delivery), and the unhealthy class tag indicates samples sourced from pregnant women who delivered a "preterm" baby (or babies) prior to completing 37 weeks of gestation.

At step 404, the training data comprises of a plurality of microbial abundance profiles corresponding to each of the collected plurality of training biological samples, wherein each microbial abundance profile corresponding to a training biological sample comprises of one or a plurality of feature (s) and respective abundance value (s) of the feature (s), wherein each feature in the microbial abundance profile corresponds to one of a plurality of microbial taxonomic groups present in the plurality of training biological samples.

In the next step 406, the training data (*TR*) is randomly partitioned into two sets - namely, an internal-train (*ITR*) and an internal-test (*ITS*), based on a parameter '$L_1$', wherein $L_1$% training biological samples from the total training data constitute the *ITR* set and (100 - $L_1$) % of the training biological samples constitute the *ITS* set. Furthermore, the random partitioning into *ITR* and *ITS* sets is performed using a stratified sampling approach with the intent of preserving the relative proportion of training biological samples belonging to the healthy class (A) or the unhealthy class (B) in the total training data in these newly drawn subsets.

In the next step 408, a predefined number of subsets are randomly selected out of the internal training set based on a parameter ($L_2$). Each of the subset comprises a randomly selected plurality of microbial abundance profiles corresponding to the plurality of training biological samples in the randomly selected subset, and wherein each of the subset comprises a proportionate part of training biological samples belonging to the healthy class (A) and the remaining training biological samples belonging to the unhealthy class (B). Thus, from *ITR, 'M'* randomly drawn subsets $ITRS_i$ (e.g., $ITRS_1$, $ITRS_2$, $ITRS_3$ .... $ITRS_M$), each containing *S* training biological samples are further generated, wherein $S = L_2$ % of the training biological samples present in *ITR*. For example, the values of $L_2$ and *M* are 80 % and 100 respectively.

In the next step 410, for each selected subset, a distribution of the abundance values of each of the features across the plurality of training biological samples in the selected subset, and the distribution of the abundance values of each of the features across the training biological samples belonging to the healthy class (A) in the selected subset and the training biological samples belonging to the unhealthy class (B) in the selected subset are noted. Thus, from each subset $ITRS_i$ (where *i* = 1, 2, 3,..., *M*), wherein there are total *S* training biological samples, each of which are described by *N* features ($F_j$) (where *j* = 1, 2, 3, ..., *N*), the distributions of each of the features ($ITRS_iDF_j$) across *S* training biological samples are noted. Similarly, from each subset $ITRS_i$, wherein there are $S_A$ training biological samples belonging to the healthy class (A) and $S_B$ training biological samples belonging to the unhealthy class (B), each of the training biological samples being described by *N* features ($F_j$; *j* = 1, 2, 3, ..., *N*), the distributions of each of the features ($ITRS_iD_AF_j$) across $S_A$ training biological samples, and the distributions of each of the features ($ITRS_iD_BF_j$) across $S_B$ training biological samples are noted.

In the next step 412, from the noted distributions of each selected subset, a first quartile value (*Q1*) and a third quartile value (*Q3*) of the distribution of each of the features is calculated across each of the plurality of training biological samples in the selected subset. In an example, the respective first quartile value (*Q1*) and the third quartile value (*Q3*) of $ITRS_iDF_j$ may also be referred $Q1ITRS_iDF_j$ and $Q3ITRS_iDF_j$.

Furthermore, in the next step 414, for each selected subset, a second quartile value of the distribution of each of the features across the training biological samples belonging to the healthy class ($Q2_A$) in the selected subset and the training biological samples belonging to the unhealthy class ($Q2_B$) in the selected subset is calculated. Thus, in an example, the median value (in other words, the second quartile value) of ($ITRS_iD_AF_j$) is referred as $Q2ITRS_iD_AF_j$, and the median value of ($ITRS_iD_BF_j$) is referred as $Q2ITRS_iD_BF_j$.

In the next step 416, for the M subsets of $ITRS_j$, a total of M values for each of $Q1ITRS_iDF_j$, $Q3ITRS_iDF_j$, $Q2ITRS_iD_AF_j$, and $Q2ITRS_iD_BF_j$, are calculated. Further at step 418, median value ( $\widetilde{Q1}_J$ ) is calculated for all calculated Q1, median value ( $\widetilde{Q3}_J$ ) is calculated for all calculated Q3, median value ( $\widetilde{Q2_A}_J$ ) is calculated for all calculated $Q2_A$ and median value ( $\widetilde{Q2_B}_J$ ) is calculated for all calculated $Q2_B$. Thus, $\widetilde{Q1}_J$ = median of {$Q1ITRS_1DF_j$, $Q1ITRS_2DF_j$, $Q1ITRS_3DF_j$,... $Q1ITRS_MDF_j$} $\widetilde{Q3}_J$ = median of {$Q3ITRS_1DF_j$, $Q3ITRS_2DF_j$, $Q3ITRS_3DF_j$,... $Q3ITRS_MDF_j$} $\widetilde{Q2_A}_J$ = median{$Q2ITRS_1D_AF_j$, $Q2ITRS_2D_AF_j$, $Q2ITRS_3D_AF_j$, .... $Q2ITRS_MD_AF_j$} $\widetilde{Q2_B}_J$ = median {$Q2ITRS_1D_BF_j$, $Q2ITRS_2D_BF_j$, $Q2ITRS_3D_BF_j$, .... $Q2ITRS_MD_BF_j$} (where *i* = 1,2,3, ..., *M*; and *j* = 1,2,3, ...,*N*)

In the next step 420, a Mann-Whitney test is performed to test if a value of the feature ($F_j$) is significantly ($p<0.1$) different between the training biological samples belonging to the healthy class ($S_A$) and the training biological samples belonging to the unhealthy class ($S_B$) in each of the M randomly drawn subsets $ITRS_j$. Other statistical tests based on the nature of distribution (e.g., t-test for normal distribution), nature of sampling (e.g., Wilcoxon signed rank test for paired case and control samples) or other methods of statistical comparison relevant for microbiome datasets (e.g., ALDEx2) can also be adopted.

In the next step 422, the features are shortlisted based on a first predefined criteria utilizing calculated median values and the Mann-Whitney test. The first predefined criteria comprises if a feature $F_j$) is observed to have significantly (p<0.1) different values in $S_A$ compared to $S_B$ in more than 70% of M subsets, and if $\widetilde{Q2_{A_J}} >= Q2_{min}$ OR

$\widetilde{Q2_{B_J}} >= Q2_{min}$ (a pre-defined feature 'abundance' threshold and $Q2_{min}$ threshold as described in the case study). $F_j$ is added to a set of shortlisted features ($SF$).

In the next step 424, a set of features is generated using the shortlisted features ($SF$) using a second predefined criteria, wherein the set of features are less than or equal to 15. If the number of shortlisted features ($SF$) obtained in previous step satisfies the criteria $1 \leq SF \leq 15$, then the training process proceeds to model building with all the features in $SF$. If no shortlisted features ($SF$) are obtained in previous (i.e., $SF < 1$) then following step is performed with all the features $F_j$ for evaluating the ability of the features, when considered independently, to distinguish between training biological samples belonging to the healthy class (A) and the unhealthy class (B). Similarly, if the number of shortlisted features ($SF$) obtained in previous step exceeds fifteen ($SF > 15$) then following step is performed with all the shortlisted features (SF) for evaluating the ability of the features, when considered independently, to distinguish between the training biological samples belonging to the healthy class (A) and the unhealthy class (B).

Steps for shortlisting the features in case of $SF < 1$ or $SF > 15$: For each of the features (obtained previously) taken individually, different threshold values are used to classify the samples belonging to the set *ITR*, and the results are cumulated to construct a receiver operating characteristic curve (ROC curve) for each of the features. The area under the curve (AUC) of the ROC curve of any feature ($AUC^F$) is indicative of the utility of the feature to distinguish between training biological samples belonging to the healthy class (A) and the unhealthy class (B), and the same is computed for every feature. The shortlisted features ($SF$) set is modified to include only the top fifteen features from a list of features arranged in a descending order of the $AUC^F$ values.

In the next step 426, a plurality of combinations of the features present in the set of features is created to generate corresponding plurality of candidate feature sets ($CF$), wherein the plurality of combinations of features comprises a minimum of one and a maximum of 15 features. In an embodiment, the maximum possible candidate feature sets that can be created in this process is $K = 2^{15} - 1 = 32767$ (i.e., maximum value of K = 32767).

In the next step 428, a plurality of candidate models is built corresponding to each of the plurality of candidate feature sets. At step 430, a model evaluation score ($MES$) is calculated corresponding to each of the plurality of candidate models. For each candidate feature set $CF_K$, a corresponding candidate model $CM_K$ is built and evaluated as mentioned in the steps mentioned below.

Steps for evaluating the candidate model:

• Step 1: The values of the features $F_j$ constituting a candidate feature set defining the training biological samples in *ITR* are transformed to $F_j'$ such that - $\widetilde{Q1_j}$, $\widetilde{Q3_j}$, $\widetilde{Q2_{A_J}}$ and $\widetilde{Q2_{B_J}}$

$$F_j' = 0 \ldots\ldots\ldots\ldots\ldots \text{ if } F_j < \widetilde{Q1_j}$$

$$F_j' = 1 \ldots\ldots\ldots\ldots\ldots \text{ if } F_j > \widetilde{Q3_j}$$

$$F_j' = 0.5 \ldots\ldots\ldots\ldots\ldots \text{ if } \widetilde{Q1_j} = \widetilde{Q3_j}$$

$$F_j' = \frac{F_j - \widetilde{Q1_j}}{\widetilde{Q3_j} - \widetilde{Q1_j}} \ldots\ldots\ldots\ldots\ldots \text{ if } \widetilde{Q1_j} < F_j < \widetilde{Q3_j}$$

• Step 2: If for a feature $F_j$, it is observed that $\widetilde{Q2_{B_J}} > \widetilde{Q2_{A_J}}$, then the feature $F_j$ is tagged as a 'numerator' feature and added to a set of numerator features $F_{numerator}$. Else, feature $F_j$ is tagged as a 'denominator' feature and added to a set of denominator features $F_{denominator}$.

• Step 3: Each candidate model ($CM_K$) is constituted as a simple ratio function given below -

$$CM_K = \frac{\Sigma F_{numerator}}{\Sigma F_{denominator}} \ldots \text{ when } F_{numerator} > 0 \text{ and } F_{denominator} > 0$$

or,

$$CM_K = \frac{\Sigma F_{numerator} + 1}{\Sigma F_{denominator} + 1} \ldots \text{ when either } F_{numerator} \text{ or } F_{denominator} = 0$$

wherein, $\Sigma F_{numerator}$ represents the sum of values of all numerator features for a particular sample, and, wherein, $\Sigma F_{denominator}$ represents the sum of values of all denominator features for a particular sample.

For each of the features, a transformed value $F'_j$ as obtained above is used in the candidate model equation.

- Step 4: A candidate model c is used to generate candidate model scores ($CMS_K$) for each of the samples in the set *ITR*. From the set of scores $CMS_K$, the top 10 percentile and bottom 10 percentile scores are removed as outliers and thereafter the maximum and minimum scores from the set $CMS_K$ are noted as $CMS_{K_{max}}$ and $CMS_{K_{min}}$ respectively.

- Step 5: Considering each of the scores in the set $CMS_K$ as a threshold (T), the model $CM_K$ is used to (re)classify the samples in the training set (*ITR.*) such that -

- the training biological sample is classified into the healthy class (A) if *CMS* > = *T*

- or the training biological sample is classified into the unhealthy class (B) if *CMS* < *T*

and based on a comparison of these classifications and the true/original classes of the training biological samples, Matthew's correlation coefficients (*MCC*) for each of the thresholds are calculated, to evaluate how well each of the thresholds can distinguish between training biological samples between the healthy class (A) and the unhealthy class (B).

- Step 6: The threshold ($T_{max}$) which provides the maximum absolute *MCC* value ($|MCC_{max}|$) is noted. If $|MCC_{max}| <$ 0.4 for a candidate model $CM_K$, then the candidate model is discarded from further evaluation. Else, the $|MCC_{max}|$ value is considered as the 'train- *MCC*' value ($|MCC_{train}|$) for the model *ITS* and the model and its corresponding $T_{max}$ threshold is used to classify the training biological samples in the internal-test set (*ITS*). In another implementation of the process, the $MCC_{max}$ threshold may not be applied for retaining the candidate model for subsequent evaluation. Before classifying the training biological samples in the *ITS* set, the values of features characterizing the training biological samples of the *ITS* set are transformed using the method mentioned in step 418 while using the earlier obtained values of $\widetilde{Q1}_J$ , $\widetilde{Q3}_J$, $\widetilde{Q2_A}_J$ and $\widetilde{Q2_B}_J$ from the *ITR* set.
- Step 7: The classification results on the training biological samples from the *ITS* set are compared against the true/original classes of the samples (with pre-assigned labels), and the *MCC* for the model $CM_K$ and its corresponding $T_{max}$ threshold on the *ITS* samples is calculated ($MCC_{test}$).
- Step 8: A model evaluation score (*MES*) for candidate model $CM_K$ is calculated as $MES = |(MCC_{train} + MCC_{test})| - |(MCC_{train} - MCC_{test})|$

In the next step 432, the model $CM_K$ is tagged as a "strong model" if all the features in the corresponding candidate feature set satisfies the Mann-Whitney test based shortlisting criteria described above. Otherwise, if any of the features in the corresponding feature set fails to satisfy the Mann-Whitney test, the model $CM_K$ is tagged as a "weak model".

Further, the above process is repeated for candidate models and respective *MES* scores are used to rank all the models. The best model is subsequently chosen based on the *MES* score. In case there are more than one model with the best *MES* score, the best model is chosen based on the following criteria (in order of preference):

(a) the model with fewer number of features (i.e., based on a smaller candidate feature set) is chosen.

(b) the model with lower $T_{max}$ (threshold value) is chosen.

Further, the best model obtained through above steps is tagged as a forward model ($MD_{fwd}$). The model $MD_{fwd}$ additionally constitutes its corresponding $T_{max}$ threshold, the $CMS_{Kmax}$ and $CMS_{Kmin}$ values, and the $\widetilde{Q1}_J$, $\widetilde{Q3}_J$, $\widetilde{Q2_{A_J}}$ and $\widetilde{Q2_{B_J}}$ values corresponding to the *ITR* set.

In the next step 434, the tags assigned to the healthy class (A) and the unhealthy class (B) of the plurality of samples present in the training data are swapped. At step 436, all of the above steps 404 to 432 to determine the best model are repeated after swapping the class labels (A <-> B) for the entire training set (TR) to obtain a best model tagged as the reverse model ($MD_{rev}$). The reverse model ($MD_{rev}$) additionally constitutes its corresponding $T_{max}$ threshold, the $CMS_{Kmax}$ and $CMS_{Kmin}$ values, and the $\widetilde{Q1}_J$, $\widetilde{Q3}_J$, $\widetilde{Q2_{A_J}}$ and $\widetilde{Q2_{B_J}}$ values corresponding to the *ITR* set.

At step 438, a plurality of forward models and a plurality of reverse models are generated by repeating step (404) through (436) for a predefined number of times using randomly partitioned internal training set and the internal test set. The steps (404) through (436) are iterated 'R' times using multiple randomly partitioned *ITR* and *ITS* sets generated initially. After each iteration, (i) the features constituting the models $MD_{fwd}$ and the models $MD_{rev}$ obtained in the current iteration (r) are compared against, and if necessary, appended to, a set of unique features $F_{unq}$ that consists of respective features constituting the $MD_{fwd}$ and $MD_{rev}$ obtained in earlier iterations (i.e., up to iteration $r$ - 1). After 'R' iterations, a plurality of forward models and a plurality of reverse models are generated for a predefined number of times using randomly partitioned internal training set and the internal test set. The iterations proceed while the value of R satisfies the following criteria -

(i) $R \leq R_{max}$
(ii) ($|F_{unq}|$ after iteration R) > ($|F_{unq}|$ after iteration R - $R_{unq}$)
(iii) $|F_{unq}|$ after iteration no. $R <= Fet_{max}$

Wherein, $R_{max}$ is a parameter indicating the maximum number of iterations allowed;
$R_{unq}$ is a parameter indicating the maximum number of iterations allowed without any cumulative increase in the number of unique features $|F_{unq}|$ in the models being generated in consecutive iterations; and
$Fet_{max}$ is a parameter indicating the maximum allowed value of $|F_{unq}|$ (i.e., the no. of unique features cumulated through the iterative process).

In an embodiment, the exemplary values of $R_{max}$, $R_{unq}$, and $Fet_{max}$ are 100, 10, 100 respectively for the present disclosure. Other values of these and other parameters here for finetuning and suitability for other datasets are within the scope of the present invention.

In the next step at 440, an ensemble of forward models is generated using the plurality of forward models and an ensemble of reverse models is generated using the plurality of reverse models. This is referred as an ensemble of forward models (ENS-$MD_{fwd}$)) and an ensemble of reverse models (ENS-$MD_{rev}$).

At step 442, the best models from each of these ensembles, i.e., the best of the forward models ($BMD_{fwd}$) and the best of the reverse models ($BMD_{rev}$) respectively, are identified.

If all models in an ensemble are weak models, the best model from the ensemble ($BMD$) is chosen by ranking the models based on their model evaluation scores and associated criteria. Also, if an ensemble contains more than one strong model, then only those strong models are considered for ranking based on their model evaluation scores and associated criteria as mentioned above, and the best model from the ensemble ($BMD$) is thereby chosen.

In the next step 444, a final single model ($FM_{single}$) is chosen as the ensemble classification model from amongst the best forward model and the best reverse model based on how they classify the individual samples from the training data. Once the best models from each of the ensemble of forward models and the ensemble of reverse models, i.e., the best of the forward models ($BMD_{fwd}$) and the best of the reverse models ($BMD_{rev}$) are identified, the final single model ($FM_{single}$) is chosen from amongst $BMD_{fwd}$ and $BMD_{rev}$ based on how well they can classify the individual training biological samples from the entire training set (TR). The AUC value for ROC curves for each of these two models are computed based on the predicted model scores for the training set (TR) samples and their pre-assigned classes (the healthy class (A) and the unhealthy class (B)). The model having the best AUC for ROC value is selected as the final single model ($FM_{single}$). If both $BMD_{fwd}$ and $BMD_{rev}$ have the same AUC value, $BMD_{fwd}$ is chosen as $FM_{single}$.

In an alternate implementation $FM_{single}$ can be chosen based whether $BMD_{fwd}$ or $BMD_{rev}$ obtains a higher *MCC* value while classifying the TR samples. Once the $FM_{single}$ model has been chosen, for classification of any samples from a test set (TS) or any sample data received during actual deployment, the $FM_{single}$ model is used after:

(a) appropriately transforming the features corresponding to the training biological sample being classified using the $\widetilde{Q1}_J$, $\widetilde{Q3}_J$, $\widetilde{Q2_{A_J}}$ and $\widetilde{Q2_{B_J}}$ values corresponding to the $FM_{single}$ model,

(b) limiting the model score between a maximum of $CMS_{K_{max}}$ and a minimum of $CMS_{K_{min}}$ alues corresponding to the $FM_{single}$ model, and

(c) classification based on the model score using its corresponding threshold $T_{max}$.

According to an embodiment of the disclosure, the ensemble of forward models (ENS-$MD_{fwd}$) and the ensemble of reverse models (ENS-$MD_{rev}$) are also evaluated for their collective classification efficiencies using an ensemble model scoring. In the ensemble scoring method, each of the models (MD) constituting an ensemble (ENS) are used to generate a model score (MS) for each of the samples from the entire TR set. For any specific training biological sample, the values of the features corresponding to the training biological sample are appropriately transformed using the $\widetilde{Q1}_J$, $\widetilde{Q3}_J$, $\widetilde{Q2_{A_J}}$ and $\widetilde{Q2_{B_J}}$ values corresponding to the model MD. The model scores (MS) are then transformed into scaled model scores (SMS) having values between -1 and +1, using the following procedure:

$$SMS = (MS - T_{max})/(CMS_{K_{max}} - T_{max}), \; ..... \; \text{when } MS >= T_{max},$$

and

$$SMS = (MS - T_{max})/(T_{max} - CMS_{K_{min}}), \; ..... \; \text{when } MS < T_{max},$$

Wherein, $T_{max}$, $CMS_{K_{max}}$, and $CMS_{K_{min}}$ values corresponding to the respective model is used.

Let $SMS_{avg}$ be the average of all SMS obtained using all models in ENS for a particular sample.

When using Forward model [ENS - $MD_{fwd}$],

$$SMS_{avg} = SMS_{avg} * (+1)$$

If $SMS_{avg} >= 0$, sample is classified as the unhealthy class 'B'
If $SMS_{avg} < 0$, sample is classified as the healthy class 'A'
When using Reverse model [ENS - $MD_{rev}$]:

$$SMS_{avg} = SMS_{avg} * (-1)$$

If $SMS_{avg} > 0$, sample is classified as the unhealthy class 'B'
If $SMS_{avg} <= 0$, sample is classified as the healthy class 'A'

If all models in one of the ensembles are weak models, then the other one having (one or more) strong models is selected as a final ensemble model ($FM_{ens}$), and subsequently used for classification of any of training biological samples from a test set (TS) or any sample data received during actual deployment of the method, using the scoring and classification process mentioned in above paragraph. If both ensembles have constituent strong models, then both the ensembles are evaluated for their efficiency by scoring them on all individual samples in TR. The AUC value for ROC curves for each of these two ensembles are computed based on the predicted $SMS_{avg}$ for all the training set (TR) samples and their pre-assigned classes. The ensemble of models having the best AUC for ROC value is selected as the final ensemble model ($FM_{ens}$). In case both ENS-$MD_{fwd}$ and ENS-$MD_{rev}$ exhibit equal AUC values then ENS-$MD_{fwd}$ is chosen as the final ensemble model ($FM_{ens}$). In an alternate implementation, $FM_{ens}$ can be chosen based whether ENS-$MD_{fwd}$ and ENS-$MD_{rev}$ obtains a higher average MCC value for their respective constituent models while classifying the *TR* samples.

Thus, either the $FM_{single}$ model or $FM_{ens}$ ensemble of models can be used for classification of any of training biological samples from a test set (*TS*) or any training biological sample data received during actual deployment.

In an embodiment, one or more predetermined microbial marker sequences out of the plurality of predetermined microbial marker sequences associated with the stool sample, are common to the first multiplexed qPCR run (Run 1), the second multiplexed qPCR run (Run 2), the third multiplexed qPCR run (Run 3), the fourth multiplexed qPCR run (Run 4), and the fifth multiplexed qPCR run (Run 5) for determining the associated quantitative abundance. In an embodiment, the one or more predetermined microbial marker sequences that are common to the first multiplexed qPCR run (Run 1), the second multiplexed qPCR run (Run 2), the third multiplexed qPCR run (Run 3), the fourth multiplexed qPCR run (Run 4), and the fifth multiplexed qPCR run (Run 5) are determined based on (i) a median abundance (obtained from microbial abundance data) of each of the plurality of predetermined microbial marker sequences obtained from the plurality of training stool samples, (ii) a frequency of occurrence of each of the plurality of predetermined microbial marker sequences constituting the ensemble ML model associated with the stool sample. More specifically, the one or more predetermined microbial marker sequences (from amongst the set of predetermined microbial marker sequences) that has/ have the highest (or relatively higher) median abundance or frequency of occurrence (as compared to the median abundance(s) or the frequency of occurrence of each microbial marker sequences in the remaining set of predetermined microbial marker sequences) across the plurality of training stool samples is/ are common to the first multiplexed qPCR run (Run 1), the second multiplexed qPCR run (Run 2), the third multiplexed qPCR run (Run 3), the fourth multiplexed qPCR run (Run 4), and the fifth multiplexed qPCR run (Run 5).

For example, a predetermined microbial marker sequence having a high median abundance or a high frequency of occurrence from the microbial abundance data is determined and utilized in more than one run. As shown in FIG. 3A, the predetermined microbial marker sequence Gut_seq1 is common for both the first multiplexed qPCR run (Run 1) and the second multiplexed qPCR run (Run 2). Similarly, the predetermined microbial marker sequence Gut_seq8 is also common for both the the third multiplexed qPCR run (Run 3) and the fourth multiplexed qPCR run (Run 4).

Similarly, the one or more predetermined microbial marker sequences out of the plurality of predetermined microbial marker sequences associated with the saliva sample, are common to the sixth multiplexed qPCR run (Run 6), the seventh multiplexed qPCR run (Run 7), and the eighth multiplexed qPCR run (Run 8), for determining the associated quantitative abundance. In an embodiment, the one or more predetermined microbial marker sequences that are common to the sixth multiplexed qPCR run (Run 6), the seventh multiplexed qPCR run (Run 7), and the eighth multiplexed qPCR run (Run 8) are determined based on (i) the median abundance (obtained from microbial abundance data) of each of the plurality of predetermined microbial marker sequences obtained from the plurality of training saliva samples, (ii) the frequency of occurrence of each of the plurality of predetermined microbial marker sequences constituting the ensemble ML model associated with the saliva sample. More specifically, the one or more predetermined microbial marker sequences (from amongst the set of predetermined microbial marker sequences) that has/ have the highest (or relatively higher) median abundance or frequency of occurrence (as compared to the median abundance(s) or the frequency of occurrence of each microbial marker sequences in the remaining set of predetermined microbial marker sequences) across the plurality of training saliva samples is/ are common to the sixth multiplexed qPCR run (Run 6), the seventh multiplexed qPCR run (Run 7), and the eighth multiplexed qPCR run (Run 8).

For example, the predetermined microbial marker sequence having the high median abundance or the high frequency of occurrence from the microbial abundance data is determined and utilized in more than one run. As shown in FIG. 3B, the predetermined microbial marker sequence Sal_seq2 is common for both the sixth multiplexed qPCR run (Run 6) and the seventh multiplexed qPCR run (Run 7). Similarly, the predetermined microbial marker sequence Sal_seq5 is also common for both the seventh multiplexed qPCR run (Run 7) and the eighth multiplexed qPCR run (Run 8).

In an embodiment, the quantitative abundance determination involves creating abundance or feature table and generation of the percent normalized abundance or feature table having percent normalized abundance values of the corresponding predetermined microbes or OTUs or taxas in the associated sample. In another embodiment, a Multi-colour Combinatorial Probe Coding (MCPC) qPCR or real-time PCR based measurement of abundance of the corresponding microbial OTUs or taxas can also be considered for quantification of a predefined set of taxas. Alternatively, any other pre-processing techniques or data normalization techniques known in the state of art can be used for normalization and feature selection from the main feature table.

**Design configuration & number of multiplexed qPCR runs required for quantifying the abundance of microbial marker sequences or microbes or microbial taxonomic groups or microbial taxa/ features:**

The quantitative abundance of each of the microbial marker sequences or microbes, that are common to each of the multiplexed qPCR runs (from the first multiplexed qPCR run to the fifth multiplexed qPCR run or from the sixth multiplexed qPCR run to the eighth multiplexed qPCR run), is determined based on a normalizing factor ($NF_{run}$) associated with each multiplexed qPCR run and the quantitative abundance of associated microbial marker sequence in the corresponding multiplexed qPCR run.

For example, considering a maximum of five unique DNA fragments, each representing a microbial marker sequence or taxa or spike DNA, can be quantified in a one multiplexed qPCR run. Therefore, to analyze a disease signature (captured in an ML model) comprising of 'n' marker sequence/microbial taxa/ features, a minimum of

$$(1 + \lceil (n-4)/4 \rceil)$$ multiplexed qPCR runs would be required wherein 'n' is the unique number of microbial taxonomic groups constituting the frugal set of markers, and wherein each multiplexed qPCR run is configured to determine, in the test biological sample, the relative abundance of a predetermined subset of the microbial marker sequences constituting the disease signature. This minimum number is based on assumptions that:

(a) the spike DNA should be analyzed at least once in one of the '(1 + $\lceil (n-4)/4 \rceil$)' multiplexed qPCR runs; and
(b) an overlap of at least one microbial taxa/ features was done between two corresponding runs.

For example, if a disease signature comprises of 8 microbial taxa (A, B, C, D, E, F, G, and H), then at least TWO multiplexed qPCR runs would be required, where Z is the spike DNA of known concentration and taxa 'D' is analyzed in both multiplexed qPCR runs. Here, $\lceil (n-4)/4 \rceil$ indicates a ceiling value of the expression. Thus, the minimum no. of required qPCR runs would be:

1 for 1-4 signatures/ features
2 for 5-8 signatures/ features
3 for 9-12 signatures/ features
4 for 13-16 signatures/ features, and so on...

**Example A: Run 1: Z A B C <u>D</u>; Run2: <u>D</u> E F G H**

Similarly, for a feature size of 12 (A, B, C, D, E, F, G, H, I, J, K, and L), at least THREE multiplexed qPCR runs would be required, where Z is the spike DNA of known concentration and taxa 'D' and 'H' are analyzed in twice.

**Example B: Run 1: Z A B C <u>D</u>; Run 2: <u>D</u> E F G <u>H</u>; Run 3: <u>H</u> I J K L**

If the number of features constituting the signature is not optimal for the above condition, i.e., for e.g., the number of features is 10, then more than one microbial taxon can be analyzed twice. The same is exemplified below, wherein taxa C and D are analyzed twice (in Runs 1 and 2). Similarly, taxa F and G are also analyzed twice (in Runs 2 and 3).

**Example C: Run 1: Z A B <u>C D</u>; Run 2: <u>C D</u> E <u>F G</u>; Run3: <u>F G</u> H I J**

In alternate implementations, the spike DNA (Z) can be analyzed in each of the runs. In that scenario, the first multiplexed qPCR will be able to accommodate up to FOUR features. Each additional multiplexed qPCR run will accommodate up to THREE new/ additional features as shown by underlining in the example below. Thus, two multiplexed qPCR runs would be required for a feature set of up to seven; three qPCR runs for a feature set of up to ten and so on.

**Run 1: Z A B C <u>D</u> ; Run 2: Z D <u>E F G</u> ; Run 3: Z G <u>H I J</u>**

Furthermore, if the number of features is not optimal for the above condition, then two or more taxa/ features can be analyzed multiple times as shown in example C.

**Methodology to interpret/ quantify the abundance of a microbial marker sequences or taxon or microbes or microbial taxonomic groups from data obtained from above qPCR configurations:**

Given that the concentration of the spike DNA (Z) is previously known - say $X_1$. If the measured concentration of Z in the multiplexed qPCR is $X_2$, then all the measured concentration in a single multiplexed qPCR run can be normalized multiplying by a normalizing factor ($NF_{run}$) of $X_1/X_2$.

In cases where the spike DNA is only analyzed in only one of the multiplexed qPCR runs (as shown in examples A, B and C), then the normalized values of the taxa/ feature in the first run which is/ are re-analyzed in the Run 2, can be used for adjusting the concentrations inferred from the Run 2 of the multiplexed qPCR. Following Example-A (described previously),

Actual conc of Z: $X_1$
Measured conc of Z: $X_2$
Normalizing factor $NF_{run1}$: $X_1/X_2$
Inferred conc. of A (from Run 1): $A'_{run1} \times NF_{run1}$
Inferred conc. of B (from Run 1): $B'_{run1} \times NF_{run1}$
Inferred conc. of C (from Run 1): $C'_{run1} \times NF_{run1}$
Inferred conc. of D (from Run 1): $D'_{run1} \times NF_{run1}$

Where $A'_{run1}$, $B'_{run1}$, $C'_{run1}$, and $D'_{run1}$ are the measured/ analyzed concentrations of taxa/ feature A, B, C and D respectively.
Normalizing factor $NF_{run2}$: Inferred conc. of D from Run 1/ Measured concentrations of feature D in Run 2

Inferred conc. of E: $E'_{run1} \times NF_{run2}$
Inferred conc. of F: $F'_{run1} \times NF_{run2}$
Inferred conc. of G: $G'_{run1} \times NF_{run2}$
Inferred conc. of H: $H'_{run1} \times NF_{run2}$

The same protocol may be repeated for normalizing/ adjusting the concentrations measured from all subsequent runs (as in example B). In case wherein more than once feature is analyzed in subsequent runs (as in example C), a median Normalizing factor (*NF*) - derived from the *NFs* for each of the replication features may be used for computing the inferred concentrations from that run.

In alternate implementations, wherein the spike DNA (Z) is analyzed in each of the runs (as in example D), Normalizing factor (*NF*) corresponding to each of the runs may be computed and used for inferring the concentrations of the constituent features. In cases, where the measured spike DNA (Z) concentration varies by more than 25% from the actual concentration, it is suggested that the observations from the said multiplexed qPCR run be discarded, and a fresh multiplexed qPCR run for the sub-set of features be performed.

In an alternate implementation using multiplexed qPCR runs, the marker feature (marker microbe or taxa) having the lowest variance in relative abundance in training data across both the classes, is selected as the anchor marker (*AM*), and the relative abundance of each of the markers is computed by multiplying the ratio of their estimated/inferred DNA concentrations and the estimated/inferred DNA concentration of *AM* with the median abundance of *AM* across all training data. For example, if the marker features are A, B, C and D. wherein A is the anchor marker (*AM*) having a median abundance of $ABN_{AM}$, then the abundances of the marker features B, C and D will be computed as;

$$ABN_B = (Inferred\ conc.\ of\ B / Inferred\ conc.\ of\ A) \times ABN_{AM}$$

$$ABN_C = (Inferred\ conc.\ of\ C / Inferred\ conc.\ of\ A) \times ABN_{AM}$$

$$ABN_D = (Inferred\ conc.\ of\ D / Inferred\ conc.\ of\ A) \times ABN_{AM}$$

At step 208 of the method 200, a model score is determined based on the quantitative abundance of each of the plurality of predetermined microbial marker sequences associated with the biological sample obtained at step 206 of the method 200, through the ML module 114. The ML module 114 includes a pre-determined machine learning (ML) model which is explained and obtained at step 206 of the method 200 is employed to determine the model score based on the quantitative abundance.

At step 210 of the method 200, the risk assessment of the subject is performed through the assessment module 116. The risk assessment is performed based on the model score obtained at step 208 of the method 200 and a predefined threshold value. For example, if the model score obtained at step 208 of the method 200 is greater than or equal to the predefined threshold value, then the subject is assessed to be having the risk of PTD. If the model score obtained at step 208 of the method 200 is less than the predefined threshold value, then the subject is assessed as not having the risk of the PTD.

At step 212 of the method 200, a personalized recommendation for the subject assessed as having the risk of the PTD at step 210 of the method 200 is designed through the recommendation module 118. In an embodiment, the personalized recommendation includes_utilizing the plurality of predetermined microbial marker sequences that constituting the pre-determined machine learning model to identify one or more antibiotic target candidates that ameliorate the risk of preterm delivery.

More specifically, the designing of the one (or a combination of) antibiotic candidates/ targets may be performed by mapping the features (i.e., organisms/ taxa/ representative microbial marker sequences) constituting the ML model to the complete set of microbes (or a pre-defined subset of the same) using the following steps.

At step 1, pair-wise correlations (using the Pearson's and/or spearman's correlation index) are computed between abundances of features (i.e., organisms/ taxa/ representative microbial marker sequences) constituting the ML model and the abundances corresponding to the complete set of microbial taxa (represented by microbial marker sequences) computed individually from (a) the subset of biological samples corresponding to the healthy class i.e. the class of samples that are taken from pregnant women with term delivery/ outcome and (b) the diseased class i.e. the class of samples that are taken from pregnant women with preterm delivery outcome, wherein both the samples belonging to the healthy and diseased classes are used as training data for generating the ML model.

At step 2, positive and negative interactions between features (i.e., organisms/ taxa/ microbes) constituting the ML model and all other taxa in the healthy and the diseased class of training samples (individually) are deduced using critical correlation ($r$) value as the cut-off (as taught in Batushansky et al., 2016), such that inter-taxa correlation index values greater than $+r$ value are affiliated as 'positive interactions', while those less than $-r$ value are affiliated as 'negative interactions'.

At step 3, the steps 1 and 2 are repeated 1000 times and only those interactions are considered relevant that appear in at least 70% of iterations with a BH (Benjamini-Hochberg) corrected p-value cut-off of 0.1 are retained (hereafter referred to as model taxa interactions corresponding to health and diseased class of samples).

At step 4, thereafter, following set of rules (indicated in Table 3 below) are used to arrive at the relevant therapeutic candidate using the retained model taxa interactions:

Table 3

| Probiotic Candidates | Antibiotic Candidates |
|---|---|
| $(M_H - C_T)_{HP}$ && $(M_H - C_T)_{DP}$ | $(M_H - C_A)_{HN} \| (M_H - C_A)_{DN}$ |
| $(M_H - C_T)_{DP}$ | $(M_D - C_A)_{HP} \| (M_D - C_A)_{DP}$ |
| $(M_D - C_T)_{HN}$ && $(M_D - C_T)_{DN}$ | |

From Table 3,

$M_H$ represents a model taxon having significantly higher abundance in healthy class;

$M_D$ represents a model taxon having significantly higher abundance in diseased (unhealthy) class;

$C_T$ represents a potential candidate for recommendation;

$C_A$ represents a potential antibiotic target candidate;

$M_H - C_T$ represents an interaction between a model taxon (abundant in healthy class) with a potential candidate for recommendation;

$M_D - C_T$ represents an interaction between a model taxon (abundant in diseased class) with a potential candidate for recommendation;

$M_D - C_A$ represents an interaction between a model taxon (abundant in diseased class) with a potential antibiotic target candidate;

$M_H - C_A$ represents an interaction between a model taxon (abundant in healthy class) with a potential antibiotic target candidate;

$HP$ represents a positive interaction in a healthy environment population;

$HN$ represents a negative interaction in a healthy environment population;

$DP$ represents a positive interaction in a diseased environment population; and

$DN$ represents a negative interaction in a diseased environment population.

One or more of the set of microbes constituting the identified antibiotic microbial taxa (represented by marker sequence) candidates may be recommended as targets (individually or in combination), targeted via antibiotics or other treatment methodologies that can reduce the abundance of the identified antibiotic microbial taxa (represented by the marker sequences) and result in ameliorating the risk of preterm delivery by pregnant women predicted with high risk of PTD. Furthermore, such antibiotic recommendation (as detailed above) may also help in promoting development of a healthy vaginal and gut microbiome, which (may) ameliorate the risk of preterm delivery by pregnant women predicted with high risk of PTD.

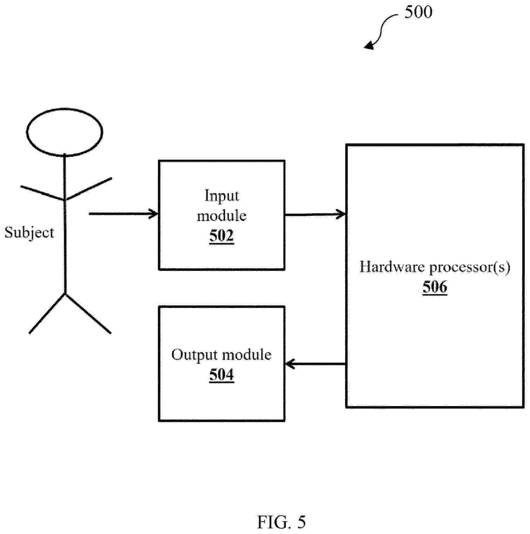

Further, a kit for risk assessment of the preterm delivery (PTD) in the subject, is disclosed. FIG. 5 illustrates an exemplary block diagram of the kit 500 for the early risk assessment of preterm delivery in the subject, according to some embodiments of the present disclosure. As shown in FIG. 5, the kit 500 includes an input module 502, one or more hardware processors 504 and an output module 506. The input module 502 is used for receiving the biological sample of the subject whose risk of PTD is to be assessed. As described, the biological sample is one out of the stool sample and the saliva sample. In an embodiment, the input module 502 may be a medium, a carrier, a set of mediums, or a set of carries that can hold the biological sample.

The one or more hardware processors 506 are configured to analyse the biological sample present in the input module 502, using the one or more steps of the method 200. In an embodiment, the one or more hardware processors 506 are equivalent or same that of the one or more hardware processors 106 of the system 100. The output module 506 is used for displaying the risk assessment of PTD in the subject, based on the analysis of the one or more hardware processors 506. In other words, the output module 506 is used for indicating on the presence or non-presence of the risk of the PTD in the subject. In an embodiment, the output module 506 includes but are not limited to a display device, an indicator, a color indicator, or any other equipment that can show the result representation on the ASD to the subject.

The embodiments of the present disclosure provides a mechanism for identifying the risk assessment of PTD in the subject by making use of the biological sample, where the biological sample is an oral microbial sample such as the stool sample and the saliva sample. Hence, the mechanism of the present disclosure is completely non-invasive for identifying the risk assessment of PTD in the subject. The present disclosure determines minimum number of microbes, or OTUs or taxonomies (in the form of the predetermined microbial marker sequences) for determining the microbial quantitative abundance using which the risk assessment of the subject for the PTD is identified. More specifically, only the fifteen microbes, or OTUs or taxonomies (represented in the form of the predetermined microbial marker sequences Gut_seq1 to Gut_seq15 of table 1) that are more influenced are identified for determining the microbial quantitative abundance of the stool sample. Similarly, only the nine microbes, or OTUs or taxonomies (represented in the form of the predetermined microbial marker sequences Sal_seq1 to Sal_seq9 of table 2) that are more influenced are identified for determining the microbial quantitative abundance of the saliva sample. Hence, the present disclosure requires less resources, simple and yet effective.

The present disclosure provides early (any time within 1st and 2nd trimester of the pregnancy) and accurate prediction or assessment regarding the risk or the predisposition to the PTD. Furthermore, this helps in providing sufficient time for the pregnant women (detected with a high risk) to take required precautionary or corrective medical advice procedure that reduce or obviate the risk of the PTD. Being the microbiome (microbes) based, the present disclosure can be used even in cases of first asymptomatic pregnancies or preterm outcomes which are not driven by vaginal infections or fetal or uterine abnormalities.

The sampling method put forward in the present disclosure is non-invasive in nature. Moreover, a single biological sample is sufficient to make an accurate diagnosis. Furthermore, given that the present disclosure necessitates the biological sample to be obtained anytime within the first and second trimesters of pregnancy, any discomfort experienced by the pregnant subjects due to the sampling procedure is expected to be significantly lower as compared to the sampling techniques employed in the prior arts, which are in some cases invasive, and are typically applicable in later stages of the pregnancy. Further, a focused, personalized and preventive recommendation is designed based on the microbes (having high influence in the corresponding biological sample). Hence, the advantages make the present disclosure as convenient and economical to deploy for mass adoption.

Example scenario:

A. Model training: The ML model along with the set of ensemble models are obtained using the data associated with the respective samples, i.e., saliva samples, or stool samples. The data associated with the respective samples is divided into training data and the test data. The present disclosure accepts data in form of a feature table for multiple observations (or samples) wherein each observation/ sample is defined by 'N' features ($F$) which are continuous variables and ($N \geq 1$). In case of training data ($TR$), each of the samples/observations further have a pre-assigned class/category which is binary in nature (e.g., A or B). In case of test data ($TS$) or data received during actual deployment of the method, the model(s) built based on training data predicts the class/category of the samples/observations.

B. Model training results: The features (microbes) of the single best model and the ensemble model are analyzed and the features that are most frequently occurred are identified, which are then used as the plurality of predetermined microbes to determine the quantitative abundance in real test cases, as explained at step 206 of the method 200.

Table 4 shows the list of features (microbes) of the ensemble model for the saliva sample along with their occurrences.

Table 4

| Unique Features in Ensemble Model | Occurrence |
|---|---|
| Sal_seq1 | 10 |
| Sal_seq2 | 9 |
| Sal_seq5 | 9 |
| Sal_seq7 | 5 |
| Sal_seq8 | 2 |
| Sal_seq3 | 2 |
| Sal_seq4 | 1 |
| Sal_seq6 | 1 |
| Sal_seq9 | 1 |

Table 5 shows an exemplary model metrics data of the single best ML model and the ensemble model that has performed the best for the saliva sample (in the 1st trimester available publicly from DiGiulio et al., PNAS, 2015).

Table 5

| Model Metrics | Single best ML Model | Ensemble ML model |
|---|---|---|
| CV (100 iterations) Mean AUC | 0.991333 | 0.994 |
| AUC Min-Max | 0.66667-1.000 | 0.93333-1 |
| AUC Std. Dev | 0.044538 | 0.01451 |
| CV (100 iterations) Mean MCC | 0.047401 | 0.810582 |
| MCC Min-Max | 0-1 | 0-1 |
| MCC Std. Dev | 0.186384 | 0.190801 |
| Training AUC | 0.997931 | 1 |
| Training MCC | 0.945517 | 0.899158 |

C. Case Study: A case study is conducted on a pregnant woman in 1st trimester for whom the risk of preterm delivery to be ascertained and the steps 1 to 8 are mentioned to explain the case study and the steps are in line with the steps of the method 200 of the present disclosure.

At step 1, the saliva sample is collected as a test sample from the pregnant woman for whom the risk of preterm delivery to be ascertained. The the saliva sample is collected during the 1st trimester of the pregnancy.

At step 2, the raw abundances of various microbial marker sequences (microbial taxonomic groups) present in the collected saliva sample are quantified. Methodology used in this step involves extraction of microbial DNA contents from the collected saliva sample followed by amplification and sequencing of either full-length or specific variable regions of the bacterial 16S rRNA marker genes using the next-generation sequencing platform or by using the multiplexed qPCR-based quantification methodology. Table 6 shows the raw abundance of various microbial marker sequences present in the collected saliva sample.

Table 6

| Microbial marker sequences | Raw abundance |
|---|---|
| Sal_seq10 | 22050 |
| Sal_seq9 | 3668 |
| Sal_seq8 | 10340 |

(continued)

| Microbial marker sequences | Raw abundance |
|---|---|
| Sal_seq7 | 5612 |
| Sal_seq6 | 2519 |
| Sal_seq4 | 4154 |
| Sal_seq11 | 6805 |
| Sal_seq12 | 3712 |
| Sal_seq1 | 88 |
| Sal_seq2 | 0 |
| Sal_seq5 | 221 |
| Sal_seq3 | 0 |
| Sal_seq13 | 7556 |
| Sal_seq14 | 1856 |
| Sal_seq15 | 751 |
| Sal_seq16 | 574 |

At step 3, the percent normalized abundances values of various microbial marker sequences are calculated using the corresponding raw abundances mentioned in Table 6. Table 7 shows the percent normalized abundances values of various microbial marker sequences present in the collected saliva sample.

Table 7

| Microbial marker sequences | Percent normalized abundances values |
|---|---|
| Sal_seq10 | 22.0504 |
| Sal_seq9 | 3.6677 |
| Sal_seq8 | 10.3403 |
| Sal_seq7 | 5.61202 |
| Sal_seq6 | 2.51878 |
| Sal_seq4 | 4.15378 |
| Sal_seq11 | 6.80513 |
| Sal_seq12 | 3.71189 |
| Sal_seq1 | 0.0883783 |
| Sal_seq2 | 0 |
| Sal_seq5 | 0.220946 |
| Sal_seq3 | 0 |
| Sal_seq13 | 7.55634 |
| Sal_seq14 | 1.85594 |
| Sal_seq15 | 0.751215 |
| Sal_seq16 | 0.574459 |

At step 4, From the normalized abundance table, abundances of only the subset of microbial marker sequences which overlap with the list of two microbial marker sequences that are provided against the ' Single best training model' are retained. Table 8 shows the model characteristics of features in the single best training model.

Table 8

| Microbial marker sequences | Sal_seq1 | Sal_seq2 | Sal_seq5 | Sal_seq3 |
|---|---|---|---|---|
| Q1 | 0 | 0 | 0 | 0 |
| Q3 | 0.034882 | 0.04095 | 0.062021 | 0.03138 |
| $Q2_A$ | 0 | 0 | 0 | 0 |
| $Q2_B$ | 0.022801 | 0.02787 | 0.030489 | 0.026593 |
| Min Model Score | 1 | | | |
| Max Model Score | 4 | | | |
| Threshold | 1.153259 | | | |
| Numerator/ Denominator | Numerator | Numerator | Numerator | Numerator |
| Model Type | Reverse | | | |

At step 5, Using Q1 and Q3 values corresponding to each training model feature in the single best training model, and the transformation is applied to the above rarefied abundances. Following are the calculated transformed abundancies:

Transformed abundance ($F_g$_Sal_seq1): 1.000
Transformed abundance ($F_g$_Sal_seq2): 0
Transformed abundance ($F_g$_Sal_seq5): 1.000
Transformed abundance ($F_g$_Sal_seq3): 0

The transformed abundance of individual features as obtained above are then used appropriately in the candidate model equation ($CM_K$) (as replicated below), and numerator and denominator sums are computed. In this case, the values obtained are as follows -

Since Numerator sum = 2.000 and Denominator sum = 0 in this case, a value of 1 is added to both numerator and denominator as per the rules:

$$\mathrm{CM_K} = \frac{\sum F_{numerator}}{\sum F_{denominator}} \; \dots \; \text{when } F_{numerator} > 0 \text{ and } F_{denominator} > 0 \text{ or,}$$

$$\mathrm{CM_K} = \frac{\sum F_{numerator}+1}{\sum F_{denominator}+1} \; \dots \; \text{when either } F_{numerator} \text{ or } F_{denominator} = 0$$

Numerator sum: 3.000
Denominator sum: 1.000

At step 6, the sample model score (MS) is computed using above Numerator sum and Denominator sum. The sample model score (MS) is then transformed into scaled model score (SMS) (having values between -1 and +1, using following rules-

$$SMS = (MS - T_{max})/(CMS_{K_{max}} - T_{max}), \; \dots \; \text{when } MS >= T_{max},$$

and

$$SMS = (MS - T_{max})/(T_{max} - CMS_{K_{min}}), \; \dots \; \text{when } MS < T_{max},$$

Wherein, $T_{max}$, $CMS_{Kmax}$, and $CMS_{Kmin}$ values corresponding to the respective model is used. For this purpose, the values of threshold: 1.153259, Maximum model score: 4.000, Minimum model score: 1.0000 for single best model (as mentioned in Table 8) are employed.

Model score ($MS$): 3.000
Scaled model score ($SMS$): 0.648721

At step 7, the $SMS$ is then used for predicting the risk of preterm birth of the individual from whom the saliva sample is obtained. Since both forward model and reverse model are evaluated, the final selected model is then used for classification or prediction). Here in this case, final selected single best model is a forward model, hence the final prediction score value is calculated as ($SMS$) * +1)

Final pred_score is -0.648721
Since the value is <0, the prediction class is "A" i.e., "Low risk of preterm delivery" Following the same series of steps, if the value of $SMS$ is greater than '0' then prediction class will be "B" and thus the risk category for the individual from whom the saliva sample is obtained will be "High risk of preterm delivery".

At step 8, similarly, for the ensemble ML model, all the steps are repeated for all the single models in the ensemble and finally the average of all the final prediction score is calculated using sample model scores ($SMS$) and the class prediction is done based on final average prediction score obtained for that sample.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein addresses unresolved problem for accurate risk assessment of the PTD delivery in the subject, by providing the early prediction (within 13 weeks of pregnancy or earlier) regarding risk or predisposition to PTD. This helps in providing sufficient time for pregnant women (detected with a high risk) to take required precautionary or corrective medical advice or procedures that reduce or obviate the risk of the PTD. The present disclosure provides the scope of complete monitoring of a pregnant woman in the first and second trimester for identification of risk of the PTD. With the present disclosure, the woman can, at any point of the pregnancy period (<27 weeks), get an assessment of the risk of the PTD.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method (200) for an early risk assessment of preterm delivery in a subject, comprising the steps of:

collecting a biological sample from the subject whose risk of preterm delivery is to be assessed (202);
extracting microbial deoxyribonucleic acid (DNA) sequences from the biological sample (204);
determining a quantitative abundance of each of a plurality of predetermined microbial marker sequences associated with the biological sample, from the extracted DNA sequences, using a set of probes specific to each of the plurality of predetermined microbial marker sequences associated with the biological sample, through a multiplexed quantitative Polymerase Chain Reaction (qPCR) technique (206);
determining, via one or more hardware processors, a model score based on the quantitative abundance of each of the plurality of predetermined microbial marker sequences associated with the biological sample, using a pre-determined machine learning (ML) model associated to the biological sample (208); and
performing, via the one or more hardware processors, the early risk assessment of preterm delivery in the subject, based on the model score and a predefined threshold value associated with the biological sample (210).

2. The method as claimed in claim 1, further comprising:
designing, a personalized recommendation for the subject assessed as having risk of preterm delivery, by utilizing a set of rules for the plurality of predetermined microbial marker sequences that constitute the pre-determined machine learning model to identify one or more personalized antibiotic target candidates that ameliorate the risk of preterm delivery (212).

3. The method (200) as claimed in claim 1, wherein the biological sample collected from the subject is one of: (i) a stool sample and (ii) a saliva sample.

4. The method (200) as claimed in claim 1, wherein the plurality of predetermined microbial marker sequences associated with the biological sample being the stool sample are listed in Table 1 comprising Gut_seq1 to Gut_seq15.

5. The method (200) as claimed in claim 1, wherein the plurality of predetermined microbial marker sequences associated with the biological sample being the saliva sample are listed in Table 2 comprising Sal_seq1 to Sal_seq9.

6. The method (200) as claimed in claim 1, wherein the set of probes specific to each of the plurality of predetermined microbial marker sequences associated with the biological sample being the stool sample are utilized in a first multiplexed qPCR run, a second multiplexed qPCR run, a third multiplexed qPCR run, a fourth multiplexed qPCR run, and a fifth multiplexed qPCR run, to determine the quantitative abundance of each of the plurality of predetermined microbial marker sequences associated with the biological sample, and wherein:

(i) the plurality of predetermined microbial marker sequences, the quantitative abundance of which are being determined through the first multiplexed qPCR run are: Gut_seq1, Gut_seq2, Gut_seq3, and Gut_seq4 listed in Table 1;
(ii) the plurality of predetermined microbial marker sequences, the quantitative abundance of which are being determined through the second multiplexed qPCR run are: Gut_seq1, Gut_seq5, Gut_seq6, and Gut_seq7 listed in Table 1;

(iii) the plurality of predetermined microbial marker sequences, the quantitative abundance of which are being determined through the third multiplexed qPCR run are: Gut_seq8, Gut_seq5, Gut_seq9, and Gut seq10 listed in Table 1;

(iv) the plurality of predetermined microbial marker sequences, the quantitative abundance of which are being determined through the fourth multiplexed qPCR run are: Gut_seq8, Gut_seq11, Gut_seq12, and Gut_seq13 listed in Table 1; and

(v) the plurality of predetermined microbial marker sequences, the quantitative abundance of which are being determined through the fifth multiplexed qPCR run are: Gut_seq6, Gut_seq11, Gut_seq14, and Gut_seq15 listed in Table 1.

7. The method (200) as claimed in claim 1, wherein the set of probes specific to each of the plurality of predetermined microbial marker sequences associated with the biological sample being the saliva sample are utilized in a sixth multiplexed qPCR run, a seventh multiplexed qPCR run, and an eighth multiplexed qPCR run, to determine the quantitative abundance of each of the plurality of predetermined microbial marker sequences associated with the biological sample, and wherein:

(i) the plurality of predetermined microbial marker sequences, the quantitative abundance of which are being determined through the sixth multiplexed qPCR run are: Sal_seq1, Sal_seq2, Sal_seq3, and Sal_seq4 listed in Table 2;

(ii) the plurality of predetermined microbial marker sequences, the quantitative abundance of which are being determined through the seventh multiplexed qPCR run are: Sal_seq4, Sal_seq2, Sal_seq5, and Sal_seq6 listed in Table 2; and

(iii) the plurality of predetermined microbial marker sequences, the quantitative abundance of which are being determined through the eighth multiplexed qPCR run are: Sal_seq7, Sal_seq8, Sal_seq5, and Sal_seq9 listed in Table 2.

8. The method (200) as claimed in claim 1, wherein the pre-determined machine learning (ML) model associated to the biological sample is an ensemble ML model that is built using a microbial marker sequence abundance data associated to a plurality of training biological samples.

9. The method (200) as claimed in claim 1, wherein the plurality of predetermined microbial marker sequences associated with the biological sample are features of the associated pre-determined machine learning (ML) model.

10. The method (200) as claimed in claim 6, wherein one or more predetermined microbial marker sequences out of the plurality of predetermined microbial marker sequences associated with the biological sample being the stool sample, are common to one or more of the first multiplexed qPCR run, the second multiplexed qPCR run, the third multiplexed qPCR run, the fourth multiplexed qPCR run, and the fifth multiplexed qPCR run for determining the quantitative abundance, and wherein the one or more predetermined microbial marker sequences that are common to the one or more of the first multiplexed qPCR run, the second multiplexed qPCR run, the third multiplexed qPCR run, the fourth multiplexed qPCR run, and the fifth multiplexed qPCR run are determined based on (i) a median abundance of each of the plurality of predetermined microbial marker sequences obtained from the associated plurality of training biological samples, and (ii) a frequency of occurrence of each of the plurality of predetermined microbial marker sequences constituting the associated ensemble ML model.

11. The method (200) as claimed in claim 7, wherein one or more predetermined microbial marker sequences out of the plurality of predetermined microbial marker sequences associated with the biological sample being the saliva sample, are common to one or more of the sixth multiplexed qPCR run, the seventh multiplexed qPCR run, and the eighth multiplexed qPCR run for determining the quantitative abundance, and wherein the one or more predetermined microbial marker sequences that are common to the one or more of the sixth multiplexed qPCR run, the seventh multiplexed qPCR run, and the eighth multiplexed qPCR run are determined based on (i) a median abundance of each of the plurality of predetermined microbial marker sequences obtained from the associated plurality of training biological samples, and (ii) a frequency of occurrence of each of the plurality of predetermined microbial marker sequences constituting the associated ensemble ML model.

12. A kit for an early risk assessment of preterm delivery in a subject, comprising:

an input module for receiving a biological sample from the subject whose risk of preterm delivery is to be assessed, wherein the biological sample of the subject is one of: (i) a stool sample and (ii) a saliva sample;

one or more hardware processors configured to analyze the biological sample using the method performed in any of the claim 1 to claim 11; and

an output module for displaying the early risk assessment of preterm delivery in the subject, based on the analysis of the one or more hardware processors.

FIG. 1

200

Collect a biological sample from the subject whose risk of preterm delivery is to be assessed **202**

Extract microbial deoxyribonucleic acid (DNA) sequences from the biological sample **204**

Determine a quantitative abundance of each of a plurality of predetermined microbial marker sequences associated with the biological sample, from the extracted DNA sequences, using a set of probes specific to each of the plurality of predetermined microbial marker sequences associated with the biological sample, through a multiplex quantitative Polymerase Chain Reaction (qPCR) technique **206**

Determine a model score based on the quantitative abundance of each of the plurality of predetermined microbial marker sequences associated with the biological sample, using a pre-determined machine learning (ML) model associated to the biological sample **208**

A

FIG. 2A

200

A

Perform the early risk assessment of preterm delivery in the subject, based on the model score and a predefined threshold value associated with the biological sample **210**

Design a personalized recommendation for the subject assessed as having risk of preterm delivery **212**

FIG. 2B

Stool sample

| Run 1 | | | | |
|---|---|---|---|---|
| Z | Gut_seq1 | Gut_seq2 | Gut_seq3 | Gut_seq4 |

| Run 2 | | | | |
|---|---|---|---|---|
| Z | Gut_seq1 | Gut_seq5 | Gut_seq6 | Gut_seq7 |

| Run 3 | | | | |
|---|---|---|---|---|
| | Gut_seq8 | Gut_seq5 | Gut_seq9 | Gut_seq10 |

| Run 4 | | | | |
|---|---|---|---|---|
| Z | Gut_seq8 | Gut_seq11 | Gut_seq12 | Gut_seq13 |

| Run 5 | | | | |
|---|---|---|---|---|
| Z | Gut_seq6 | Gut_seq11 | Gut_seq14 | Gut_seq15 |

| Z | Universal Non-specific Probe |
|---|---|

FIG. 3A

Saliva sample

FIG. 3B

400

Assign one of a healthy class tag or an unhealthy class tag to each of the samples in the collected plurality of training biological samples **402**

Generate training data comprising of a plurality of microbial abundance profiles corresponding to each of the plurality of training biological samples, wherein each microbial abundance profile corresponding to a training biological sample comprises of one or a plurality of features and respective abundance values of the features, and wherein each feature in the microbial abundance profile corresponds to one of a plurality of microbial taxonomic groups present in the training biological sample **404**

Partition the training data into an internal training set and an internal test set **406**

Randomly select a predefined number of subsets out of the internal training set, wherein each subset comprises of a randomly selected plurality of microbial abundance profiles corresponding to the training biological samples in the randomly selected subset, and wherein each subset comprises a proportionate part of samples belonging to the healthy class and the remaining samples belonging to the unhealthy class **408**

Note for each selected subset, a distribution of the abundance values of each of the features across the plurality of training biological samples in the selected subset, and the distribution of the abundance values of each of the features across the training biological samples belonging to the healthy class in the selected subset and the training biological samples belonging to the unhealthy class in the selected subset **410**

Calculate from the noted distributions of each selected subset, a first quartile value (Q1) and a third quartile value (Q3) of the distribution of each of the features across each of the plurality of training biological samples in the selected subset **412**

B

FIG. 4A

(B)

Calculate for each selected subset, a second quartile value of the distribution of each of the features across the training biological samples belonging to the healthy class (Q2_A) ) in the selected subset and the training biological samples belonging to the unhealthy class (Q2_B) in the selected subset **414**

Calculate Q1, Q3 Q2_A Q2_B for each of a predefined number of subsets (M) **416**

Calculate median value for all calculated Q1, median value for all calculated Q3, median value for all calculated Q2_A and median value for all calculated Q2_B **418**

Perform a Mann-Whitney test to test if a value of the feature is significantly different between the samples belonging to the healthy class and the samples belonging to the unhealthy class **420**

Shortlist the features based on a first predefined criteria utilizing calculated median values and the Mann-Whitney test **422**

Generate a set of features using the shortlisted features using a second predefined criteria, wherein the set of features are less than or equal to 15 **424**

Create a plurality of combinations of the features present in the set of features to generate corresponding plurality of candidate feature sets, wherein the plurality of combinations of features comprises a minimum of two and a maximum of 15 features **426**

(C)

FIG. 4B

C

Build a plurality of candidate models corresponding to each of the plurality of candidate feature sets **428**

Calculate a model evaluation score (MES) corresponding to each of the plurality of candidate models **430**

Select a model out of the plurality of candidate models as the best model using the highest model evaluation score wherein the selected model is tagged as a forward model **432**

Swap the tags assigned to the healthy class and the unhealthy class of the plurality of training biological samples present in the training data **434**

Identify a model as a reverse model by repeating the steps (**404** to **432**) for the training data obtained after swapping the tags **436**

Generate a plurality of forward models and a plurality of reverse models for a predefined number of times using randomly partitioned internal training set and the internal test set **438**

Generate an ensemble of forward models using the plurality of forward models and an ensemble of reverse models using the plurality of reverse models **440**

Identify a best forward model and a best reverse model using the model evaluation score **442**

Choose a final single model (FM_single) as the ensemble classification model from amongst the best forward model and the best reverse model based on how they classify the individua ltraining biological samples from the training data **444**

FIG. 4C

500

Subject

Input
module
**502**

Hardware processor(s)
**506**

Output module
**504**

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321028612

**Non-patent literature cited in the description**

- **DIGIULIO et al.** *PNAS,* 2015